# EUROPEAN PATENT APPLICATION

(11) **EP 4 233 918 A2**
(43) Date of publication of application: **30.08.2023**
(21) Application number: 23150094.3
(22) Date of filing: 03.01.2023
(51) Int. Cl.: A61L 2/10, A61L 9/20

(54) **INACTIVATION DEVICE FOR BACTERIA AND/OR VIRUSES**

(30) Priority: 25.02.2022 JP 2022028289
(71) Applicant: Ushio Denki Kabushiki Kaisha, Tokyo-to 100-8150 (JP)
(72) Inventor: SHIGOKU, Minoru, Tokyo, 100-8150 (JP); FUJISAWA, Shigeki, Tokyo, 100-8150 (JP)
(74) Representative: Tomerius, Isabel

(57) **Abstract**

An inactivation device (1) for bacteria and/or viruses includes: an ultraviolet light source emitting ultraviolet light having a main emission wavelength belonging to within a wavelength range from 190 nm or more to less than 240 nm; an enclosure (10) including a light-extraction surface (15) that is constituted by an opening or a window member (14), and housing the ultraviolet light source thereinside; and a reflective member (13) on which the ultraviolet light that has passed through the light-extraction surface (15) is incident and changes a traveling direction of the ultraviolet light. The reflective member (13) has a reflective surface that makes an optical axis of the ultraviolet light that has been incident change to a direction directly away from the enclosure (10), or to a direction away from the enclosure (10) after traveling toward a center area of the enclosure (10), with respect to a first direction parallel to the light-extraction surface (15).

## Description

### Technical Field

The present invention relates to an inactivation device for bacteria and/or viruses.

### Background Art

Bacteria and/or viruses present in a space or on a surface of an object is often the main cause of airborne or aerosol infections, and there is concern that the spread of infectious diseases may threaten daily life. In particular, since infectious diseases are easily spread in facilities where people frequently gather, such as medical facilities, schools, and government offices, and in vehicles such as cars, trains, buses, airplanes, and ships, effective measures for inactivating bacteria and/or viruses are needed.

The method of ultraviolet light irradiation is conventionally known as an inactivation method of bacteria and/or viruses. DNA exhibits the highest absorption characteristics near a wavelength of 260 nm. Low-pressure mercury lamps show a high emission spectrum near a wavelength of 254 nm. For this reason, the technique of sterilization using low-pressure mercury lamps has been widely used.

However, ultraviolet light in certain wavelength bands is known to pose a risk of adversely affecting human bodies when radiated to human bodies. Therefore, methods and devices of inactivating bacteria and/or viruses present in a space or on the surface of an object are being considered such that no ultraviolet light is radiated to humans.

Patent Literature 1, for example, discloses a germicidal lamp that is mounted to be hung on the ceiling in a space and emits ultraviolet light toward the ceiling in a manner that ultraviolet light emitted from the germicidal lamp is not directly radiated to persons in the space.

### Citation List

### Patent Literature

Patent Literature 1: JP-UM-A-1993-021951

### SUMMARY OF THE INVENTION

### Technical Problem

Bacteria and/or viruses generally spread from person to person, especially through the surfaces of human bodies (e.g., skin and hair), the surfaces of objects with which people frequently come into contact (e.g., desks and door knobs), and droplets or air in a space where people frequently come and go. Hence, it is desirable to irradiate the space near human bodies with ultraviolet light in order to inactivate bacteria and/or viruses.

However, as mentioned above, it is undesirable to unlimitedly irradiate the space with ultraviolet light from the viewpoint of the safety of human bodies. In this viewpoint, standards for upper limits of irradiation doses have been established in consideration of the safety of human bodies. As of the filing date of the present application, the ACGIH (American Conference of Governmental Industrial Hygienists) standard, for example, sets the threshold limit value (TLV) of ultraviolet light irradiation dose per 8 hours to human bodies in accordance with the wavelength band.

In addition, when a light source is mounted downward on the ceiling of a room, for example, the illuminance in an area directly below the light source is higher than the illuminance in an area away from the area directly below the light source. Hence, when a germicidal lamp or the like for inactivating bacteria and/or viruses is mounted downward on the ceiling of a room to irradiate the space near human bodies with ultraviolet light, the highest illuminance is in an area directly below its mounting position. Obviously, human bodies are expected to be present in the area directly below the lamp. Hence, when ultraviolet light is radiated to the space near human bodies, the irradiation dose of ultraviolet light is controlled in accordance with the above-mentioned ACGIH standard and other standards, with reference to the area of maximum illuminance, which is directly below the germicidal lamp in the present example being used as a standard. In this case, the irradiation dose of ultraviolet light is more limited in the surroundings of the area, i.e., the area at a wide-angle when a direction directly below the germicidal lamp is set to 0 degrees, thereby reducing the effects of inactivating bacteria and/or viruses.

In view of the above circumstances, the present invention provides an inactivation device for bacteria and/or viruses capable of enhancing the inactivation effect to a wide area compared with a conventional device, even when the ultraviolet light source is controlled such that its irradiation dose becomes less than the standard value specified in the standards or the like, in the case in which the ultraviolet light source is used to radiate ultraviolet light for inactivating the bacteria and/or viruses.

### Solution to the problem

An inactivation device for bacteria and/or viruses according to the present invention includes:
an ultraviolet light source emitting ultraviolet light having a main emission wavelength belonging to within a wavelength range from 190 nm or more to less than 240 nm;
an enclosure including a light-extraction surface that is constituted by an opening or a window member, and housing the ultraviolet light source thereinside; and
a reflective member on which the ultraviolet light that has passed through the light-extraction surface is incident and changes the traveling direction of the ultraviolet light,
the reflective member has a reflective surface that makes an optical axis of the ultraviolet light that has been incident change to a direction directly away from the enclosure, or to a direction away from the enclosure after traveling toward a center area of the enclosure, with respect to a first direction parallel to the light-extraction surface.

In the present specification, "main emission wavelength" refers to a wavelength band that has a relative intensity of 10% or more with respect to the peak intensity in the emission spectrum.

In the present specification, "inactivation" refers to a concept that encompasses eliminating bacteria and/or viruses or causing them to lose their infectivity or toxicity, and "bacteria" refers to microorganisms such as bacteria and fungi (mold). Hereinafter, "bacteria and/or viruses" may be collectively referred to as "germs". In addition, hereinafter, "an inactivation device for bacteria and/or viruses" may be simply abbreviated to "an inactivation device".

In the present specification, an emission angle of ultraviolet light that has passed through the light-extraction surface and emitted from the inactivation device is referenced with the optical axis of the light traveling in the direction orthogonal to the light-extraction surface. Based on this definition, the emission angle of ultraviolet light emitted from the inactivation device and traveling in a direction orthogonal to the light-extraction surface (normal direction) is 0 degrees.

In the inactivation device for bacteria and/or viruses according to the present invention, the traveling direction of the ultraviolet light emitted from the ultraviolet light source housed in the enclosure is changed by the reflective member after the ultraviolet light passes through the light-extraction surface. The reflective members reflect the ultraviolet light in the direction away from the enclosure, thereby increasing the emission angle of the ultraviolet light emitted from the inactivation device. In the conventional case, since the intensity of ultraviolet light emitted at an emission angle of 0 degrees is the maximum, the illuminance in an area irradiated with ultraviolet light at 0 degrees (hereinafter referred to as "illuminance in the 0 degrees direction" for convenience), in other words, the illuminance in an area facing to the light-extraction surface in its normal direction becomes high. However, in the case of the present invention, since the emission angle of the ultraviolet light emitted from the inactivation device increases, the intensity of the ultraviolet light emitted in the wide-angle direction in which the emission angle is large, is higher than that emitted in the 0 degrees direction, thus the illuminance in an area facing in the wide-angle direction (hereinafter referred to as "illuminance in the wide-angle direction" for convenience) becomes higher. In other words, the radiation intensity of ultraviolet light in the 0 degrees direction is lower than the radiation intensity of ultraviolet light in the wide-angle direction, thus the illuminance in the 0 degrees direction is relatively lower than the illuminance in the wide-angle direction. For this reason, when ultraviolet light is radiated to humans or a space near human bodies, even if the ultraviolet light source is turned on with an intensity such that the irradiation dose is equal to or less than the standard value specified by ACGIH, etc., the ultraviolet light is radiated in a wide area, thereby effectively inactivating bacteria and/or viruses.

According to the present invention, the angle indicating the maximum value of the radiation intensity of ultraviolet light emitted from the light-extraction surface of the inactivation device (hereinafter referred to as "peak angle") when the radiation intensity is broken down by angle exists in the wide-angle direction. The peak angle of the ultraviolet light emitted from the light-extraction surface is preferably designed to be 30 degrees or more and less than 90 degrees, and more preferably 30 degrees or more and less than 85 degrees. This enables ultraviolet light irradiation over a wide area while providing ultraviolet light irradiation to humans or the space near human bodies, thereby effectively inactivating bacteria and/or viruses present in the space.

In addition, the peak angle may be designed to be 40 degrees or more, or even 45 degrees or more, or even 50 degrees or more. This enables ultraviolet light irradiation in a wide-angle direction more proactively while still providing ultraviolet light irradiation to humans or the space near human bodies. Note that the lower limit of the angle range may be designed based on the transmittance distribution of the optical filter with respect to the incident angle when an optical filter is used as described below. Details will be described below.

The inactivation device for bacteria and/or viruses may include a light-shielding member that, of the ultraviolet light reflected on the reflective surface of the reflective member, blocks at least part of the ultraviolet light traveling toward the ultraviolet light source with respect to a second direction orthogonal to the light-extraction surface.

It is noted for the sake of clarity that, of the ultraviolet light reflected on the reflective surface of the reflective member, the ultraviolet light traveling toward the ultraviolet light source with respect to the second direction orthogonal to the light-extraction surface does not necessarily refer only to the ultraviolet light traveling toward the ultraviolet light source; it is intended to include ultraviolet light traveling toward the ultraviolet light source with respect to the second direction even though the ultraviolet light travels away from the ultraviolet light source with respect to the in-plane direction of the light-extraction surface.

When ultraviolet light for inactivating germs present in a space or on a surface of an object is considered to be used in an actual target space, this target space may have undesirable areas for ultraviolet light irradiation. In many cases, such areas are located on the opposite side of the area to which the ultraviolet light irradiation is intended with respect to the inactivation device to be mounted. For example, upon the consideration of mounting the inactivation device on a ceiling and walls to irradiate a room with ultraviolet light for inactivating bacteria and/or viruses, the irradiation dose (exposure amount) on a ceiling and walls is expected to increase when the lighting time of the ultraviolet light source becomes longer. A Ceiling and walls are generally covered with building sheets (sheet materials) such as wallpaper, which are usually made from polymer resins. Hence, when the ultraviolet light irradiation dose is not considered and the ultraviolet light irradiation dose increases, the sheet material may deteriorate due to the cutting of part of the molecular chains constituting the resin, etc., which may result in losing the aesthetic appeal of the ceiling or walls and decreasing the waterproof and dustproof functions. In contrast, by providing the light-shielding member that blocks ultraviolet light radiating toward the area at which the ultraviolet light source of the inactivation device is located, in other words, the opposite side of the area to which ultraviolet light irradiation is intended, this makes it possible to prevent the irradiation to an area at which ultraviolet light irradiation is undesirable, while providing ultraviolet light irradiation to an area to be irradiated.

The inactivation device for bacteria and/or viruses may include a diffusion member that diffuses the ultraviolet light in the optical path of the ultraviolet light reflected by the reflective surface.

By making the emission angle of the ultraviolet light widen to a wide angle using the reflective member, and then making the range of ultraviolet light irradiation further widen using the diffusion member, this enables ultraviolet light irradiation over a wider area. Furthermore, the inactivation device including the diffusion member reduces the difference in radiation intensity at each emission angle of light. Even when the emission angle of the ultraviolet light emitted from the inactivation device is widened at a wide angle, if there exists an emission direction with a significantly high radiation intensity, the radiation intensity in that emission direction can be used to limit the amount of ultraviolet light, which may result in decreasing the irradiation amount of ultraviolet light being emitted in the other directions. The inactivation device including the diffusion member makes the emission direction of ultraviolet light emitted from the inactivation device with a significantly high radiation intensity less likely to appear, thereby effectively providing the ultraviolet light irradiation in the space or onto the surface of an object.

The inactivation device for bacteria and/or viruses may include an optical control member that directs the ultraviolet light emitted from the ultraviolet light source in a second direction, and that is disposed between the light-extraction surface and the ultraviolet light source in the optical path of the ultraviolet light emitted from the ultraviolet light source.

The light emitted from the ultraviolet light source travels with a certain divergence angle; however, the ultraviolet light is made to be directed by the optical control member, thus most of the ultraviolet light can be incident at smaller incident angles onto the light-extraction surface. As a result, most of the ultraviolet light that has passed through the light-extraction surface is introduced to the position of the reflective member and enters the reflective member. This makes it easy to control the widening of the emission angle of ultraviolet light. Examples of the optical control member include an optical lens such as a collimator lens, and a concave or tapered reflective surface. When the ultraviolet light source is a solid-state light source such as an LED element, for example, an optical lens such as a collimator lens can be suitably used. When the ultraviolet light source is a lamp including a tube filled with luminescent gas, a block having a tapered reflective surface disposed between the tube and the light-extraction surface can be suitably used. In the latter case, when made of metal, this block itself can also serve as an electrode for the lamp. It may also be in the form of a combination of an optical lens and a concave or tapered reflective surface.

The inactivation device for bacteria and/or viruses may include an optical filter that is provided between the ultraviolet light source and the reflective member. The optical filter may transmit ultraviolet light having a wavelength belonging to within a wavelength range from 190 nm or more to less than 240 nm, and may suppress the transmission of ultraviolet light having a wavelength in a range from 240 nm or more to less than 300 nm.

In the present specification, "suppress the transmission of ultraviolet light" refers to, of the ultraviolet light emitted from the ultraviolet light source, reducing the ratio of the intensity of ultraviolet light that has passed through the optical filter and having a wavelength of 240 nm or more to less than 300 nm with respect to the intensity of light having a peak wavelength within a wavelength range from 190 nm or more to less than 240 nm. In the present invention, the use of the optical filter significantly suppresses the radiation intensity of ultraviolet light emitted outside the device, and that has a wavelength of 240nm or more to less than 300nm.

In recent years, ultraviolet light belonging to within a wavelength range from 190 nm or more to less than 240 nm has been found to be much less harmful to humans than ultraviolet light having a wavelength of 254 nm, which is considered to be a conventional germicidal light. As described above, the inactivation device is provided with an ultraviolet light source that emits ultraviolet light having a main emission wavelength belonging to within a wavelength range from 190 nm or more to less than 240 nm. However, ultraviolet light emitted from an ultraviolet light source may generally have its light output even in the wavelength range away from the peak wavelength. In the spectrum of ultraviolet light emitted from KrCI excimer lamps, for example, the light output is centered at or near the main peak wavelength of 222nm; however, the emission of ultraviolet light having a wavelength range from 240nm or more to less than 300nm, which may adversely affect human bodies, is often observed although the light intensity is extremely low compared with the intensity at the peak wavelength. Even when the inactivation device is provided with an ultraviolet light source that has the above-mentioned emission spectrum, the optical filter described above prevents the transmission of the ultraviolet light emitted from the ultraviolet light source with a wavelength of 240 nm or more to 300 nm or less, thereby reducing the amount of ultraviolet light emitted outside the device with this wavelength range. Hence, the amount of ultraviolet rays in this wavelength range that is extracted outside is reduced. Therefore, this configuration provides ultraviolet light irradiation to a space with further suppression of the adverse effects on human bodies. It is obviously noted that it is desirable to comply with safety standards on ultraviolet light irradiation dose even for ultraviolet light having a wavelength range that is significantly less harmful to human bodies.

The optical filter may be disposed between the optical control member and the above-mentioned reflective member. Although traveling with a certain divergence angle, ultraviolet light is made to be directed by the optical control member, thus most of the ultraviolet light can be incident on the optical filter at smaller incident angles. Optical filters have characteristics called incident angle dependency, in which the transparency and reflectance of each wavelength change with the incident angle of ultraviolet light incident thereon. In detail, when the incident angle to the optical filter increases, the transmittance decreases, and the reflectance increases in the wavelength band from 190 nm or more to less than 240 nm. This may pose a concern of decreasing the extraction efficiency of ultraviolet light to the outside of the inactivation device, depending on the incident angle of the ultraviolet light to the optical filter. Hence, providing the optical control member described above makes ultraviolet light become easier to be incident on the optical filter at smaller incident angles, thus the light-extraction efficiency is less likely to decrease.

In the inactivation device for bacteria and/or viruses according to the present invention, ultraviolet light with an emission angle of wider than 0 degrees at the light-extraction surface has a higher radiation intensity than ultraviolet light with that of 0 degrees.

As mentioned above, the transmittance of an optical filter depends on the incident angle of ultraviolet light. In detail, the transmittance exhibits its maximum value when the incident angle of light to the optical filter is 0 degrees, i.e. when the light is incident perpendicular to the optical filter, and the value of transmittance decreases as the incident angle increases. Hence, the angular distribution of the radiation intensity, which is constituted by a breakdown of the radiation intensity of ultraviolet light emitted from the light-extraction surface of the inactivation device per angle, can also be designed based on the transmittance distribution of the optical filter mounted in the inactivation device with respect to the incident angle.

When ultraviolet light from the ultraviolet light source is incident on the optical filter, the transmittance distribution with respect to the incident angle of the optical filter is obtained by breaking down the radiation intensity before passing through the optical filter and the radiation intensity after passing through the optical filter per incident angle and dividing the latter by the former. In the transmittance distribution with respect to the incident angle obtained in this manner, the transmittance exhibits a maximum value at an incident angle of 0 degrees, and the transmittance decreases gradually as the incident angle increases, as described above. In this transmittance distribution with respect to the incident angle, let T₀ be the value of transmittance at an incident angle of 0 degrees (i.e., the maximum value of transmittance), then the incident angle at which the transmittance exhibits the half value of To (T_{z}) (here referred to as "reference angle Z") is derived. The inactivation device may be designed such that the above-mentioned peak angle in the inactivation device is greater than or equal to this reference angle Z. Note that for deriving this reference angle Z, for example, adopted can be the analysis result on light with the highest light intensity in a wavelength range from 190 nm or more to less than 240nm, of the light emitted from the ultraviolet light source incident on the optical filter at an incident angle of 0° and passing through the optical filter.

This configuration is capable of increasing the radiation intensity of ultraviolet light in the direction of a wider emission angle compared with the case in which ultraviolet light is emitted only through the optical filter.

The upper limit of the peak angle of relative radiation intensity with respect to the emission angle is preferably less than 85 degrees as described above, or even less than 80 degrees. This enables the ultraviolet light irradiation to the space while suppressing the component of ultraviolet light traveling toward the side where the ultraviolet light source inactivation device, i.e., the opposite side of the area where ultraviolet light irradiation is intended.

The reflective surface may include a side face of a member having a conical shape, a frustum shape, or a curved rotational body shape.

The ultraviolet light source includes a plurality of LED elements; and the inactivation device for bacteria and/or viruses includes, when viewed from a second direction orthogonal to the light-extraction surface, a first area with a relatively low arrangement density or no arrangement of the LED elements, and a second area with a relatively higher arrangement density of the LED elements than that of the first area, and the second area being arranged to surround the first area.

In the present specification, "arrangement density of LED elements" may be defined as the number of LED elements mounted per unit area when each LED element has the same size, or may be defined as the area of the light-emitting surface of LED elements mounted per unit area when some of LED elements have different sizes.

When the ultraviolet light source includes a plurality of LED elements, the arrangement density can be made to vary depending on the area. The LED elements arranged in the first area may have cases in which ultraviolet light is not substantially extracted to the outside depending on the shape and position of the reflective member. For this reason, no LED element may be mounted in the first area or the arrangement density thereof may be reduced.

This configuration allows the illuminance of the ultraviolet light radiated to the area facing the first area in the second direction to become relatively lower than the illuminance of the ultraviolet light radiated to surrounding areas. As a result, the circumstances described above can, when inactivation devices are mounted on the ceiling, for example, further relax the limitations on the output and irradiation time of the ultraviolet light source, which is required to comply with the standard of irradiation dose specified by the ACGIH.

The first area may be an area where no LED element is disposed, and the light-extraction surface may be a surface facing the second area when viewed in the second direction and may have an annular shape.

The inactivation device for bacteria and/or viruses may include a first light-shielding member that blocks, of the ultraviolet light reflected on the reflective surface of the reflective member, at least part of the ultraviolet light traveling toward the ultraviolet light source with respect to the second direction; and a second light-shielding member that blocks at least part of the ultraviolet light traveling away from the ultraviolet light source with respect to the second direction.

The discussion similar to that for the above-mentioned light-shielding member can be made for the first light-shielding member. Providing the first light-shielding member in the inactivation device enables the ultraviolet light irradiation to an area to be irradiated while preventing irradiation to an area where ultraviolet light irradiation is undesired. In addition, the ultraviolet light traveling away from the ultraviolet light source with respect to the second direction can be considered separately in the first area and the second area. First, by providing the second light-shielding member that blocks the first area, when a visible light source is mounted on the opposite side of the ultraviolet light source with respect to the second light-shielding member, for example, this makes it possible to use the first area as an area for visible light illumination while performing ultraviolet light irradiation from the second area. In this case, the inactivation device also serves as an illumination device. On the other hand, in the second area, the above-mentioned reflective members expect to change the traveling direction of most of the ultraviolet light emitted mainly in the second direction, but some of the ultraviolet light that transmits the reflective member without being reflected by it is expected to travel in the second direction. Blocking such ultraviolet light can reduce the radiation intensity in the near 0 degrees direction, preferentially enabling the ultraviolet light irradiation in the wide-angle direction.

The reflective member may be arranged to surround the first area when viewed from the second direction.

The inactivation device for bacteria and/or viruses may include a diffusion member that diffuses the ultraviolet light that has been reflected on the reflective surface. The reflective surface may be formed to make the optical axis of the ultraviolet light incident thereon change in a direction directly away from the enclosure with respect to the first direction, and the diffusion member may be arranged to surround the outside of the reflective member when viewed from the second direction.

The inactivation device for bacteria and/or viruses may include a diffusion member that diffuses the ultraviolet light that has been reflected on the reflective surface. The reflective surface may be formed to make the optical axis of the ultraviolet light incident thereon change in a direction away from the enclosure after traveling toward the center area of the enclosure in the first direction, and the diffusion member may be arranged to surround the inside of the reflective member when viewed from the second direction.

In the inactivation device for bacteria and/or viruses, the ultraviolet light source may include an excimer lamp, the inactivation device for bacteria and/or viruses may include a light-shielding member that, of the ultraviolet light reflected on the reflective surface of the reflective member, blocks at least part of the ultraviolet light traveling toward the ultraviolet light source with respect to the second direction orthogonal to the light-extraction surface, and the light-shielding member may be arranged to surround the reflective member when viewed from the second direction.

The discussion similar to that for the above-mentioned light-shielding member can be made. Providing the light-shielding member disposed to surround the reflective member when viewed from a direction orthogonal to the light-extraction surface enables the ultraviolet light irradiation to an area to be irradiated while preventing irradiation to an area where ultraviolet light irradiation is undesired.

The inactivation device for bacteria and/or viruses may include a diffusion member that diffuses the ultraviolet light that has been reflected on the reflective surface, and the diffusion member may be arranged to surround the outside of the reflective member when viewed from the second direction.

The discussion similar to that for the above-mentioned diffusion members can be made. By making the emission angle of the ultraviolet light widen to a wide angle using the reflective member, and then making the range of ultraviolet light irradiation further widen using the diffusion member, this enables ultraviolet light irradiation over a wider area. Furthermore, the diffusion member also provides the effect of making an emission angle with a significantly high radiation intensity less likely to appear in the ultraviolet light emitted from the inactivation device.

The light-shielding member may include an opening through which ultraviolet light emitted from the light-extraction surface passes, and the enclosure may be arranged such that the opening and the light-extraction surface do not overlap when viewed from the second direction.

The light-shielding member may include a supporter that supports the reflective member, and the ultraviolet light may transmit between the supporter and the inner side face of the light-shielding member.

The inactivation device for bacteria and/or viruses may include a phosphor layer that converts the ultraviolet light incident thereon into visible light and that is formed on at least part of the surface of the light-shielding member that blocks the traveling of the ultraviolet light.

As mentioned above, the light-shielding member blocks ultraviolet light traveling toward an area where ultraviolet light irradiation is undesired. Forming the phosphor layer enables the ultraviolet light to be used more effectively than the case in which the ultraviolet light is simply blocked; therefore, the inactivation device can be used to perform ultraviolet light irradiation, and simultaneously, can be utilized as an illumination device. The visible light emitted from the phosphor layer can also be used to determine whether the ultraviolet light source is lit or not. In order to further enhance visibility, a wavelength range other than white, for example, red or green can also be selected.

### Advantageous Effects of invention

The present invention is capable of enhancing the inactivation effect over a wide area compared with conventional methods, in the case that an ultraviolet light source is used to radiate ultraviolet light to inactivate bacteria and/or viruses, even when the ultraviolet light source is controlled such that the irradiation dose is equal to or less than the standard value specified in standards or the like.

### BRIEF DESCRIPTION OF DRAWINGS

FIG. 1 is a perspective view illustrating a first embodiment of an inactivation device for bacteria and/or viruses according to the present invention.
FIG. 2 is a perspective view illustrating a cross-section that is cut in a plane perpendicular to the light-extraction surface in FIG. 1.
FIG. 3 is a plan view when the cross-sectional view shown in FIG. 2 is viewed in the Z direction.
FIG. 4 is a schematic view illustrating an arrangement of LED elements when viewed from the Y direction.
FIG. 5 is an enlarged view illustrating an area near the reflective member in FIG. 3 in detail.
FIG. 6 is a graph indicating the simulation results of the relative radiation intensity with respect to the emission angle of ultraviolet light of the first embodiment according to the present invention.
FIG. 7 is a graph illustrating the characteristics of the optical filter used for the simulation.
FIG. 8 is a perspective view schematically illustrating a first variation example of the first embodiment according to the present invention.
FIG. 9 is a perspective view illustrating a cross-section cut in a plane perpendicular to the light-extraction surface in FIG. 8.
FIG. 10 is a plan view when the cross-sectional view shown in FIG. 9 is viewed in the Z direction.
FIG. 11 is an enlarged view illustrating an area near the reflective member in FIG. 10 in detail.
FIG. 12 is a graph indicating the simulation results of the relative radiation intensity with respect to the emission angle of ultraviolet light of the first variation example of the first embodiment according to the present invention.
FIG. 13 is a perspective view schematically illustrating a second variation example of the first embodiment according to the present invention.
FIG. 14 is a perspective view illustrating a cross-section that is cut in a plane perpendicular to the light-extraction surface in FIG. 13.
FIG. 15 is a plan view when the cross-sectional view shown in FIG. 14 is viewed in the Z direction.
FIG. 16 is an enlarged view illustrating an area near the reflective member in FIG. 15 in detail.
FIG. 17 is a graph indicating the simulation results of the relative radiation intensity with respect to the emission angle of ultraviolet light of the second variation example of the first embodiment according to the present invention.
FIG. 18 is a cross-sectional view of the variation example in which a visible light source is mounted in the first area of the first embodiment.
FIG. 19 is a cross-sectional view of the variation example in which a visible light source is mounted in the first area of the second variation example of the first embodiment.
FIG. 20 is a perspective view illustrating the variation example in which a visible light illumination is mounted in the first area of the first embodiment.
FIG. 21 is a perspective view illustrating a second embodiment of an inactivation device for bacteria and/or viruses according to the present invention.
FIG. 22 is a perspective view illustrating a cross-section that is cut in a plane perpendicular to the light-extraction surface in FIG. 21.
FIG. 23 is a plan view when the cross-sectional view shown in FIG. 22 is viewed in the Z direction.
FIG. 24 is an enlarged view illustrating an area near the excimer lamps in FIG. 23 in detail.
FIG. 25 is a schematic view illustrating the relationship between the enclosure and the light-shielding member when viewed from the +Y direction.
FIG. 26 is a schematic view illustrating the second embodiment according to the present invention when viewed from the +Y direction.
FIG. 27 is a graph indicating the simulation results of the relative radiation intensity with respect to the emission angle of ultraviolet light of the second embodiment according to the present invention.
FIG. 28 is a plan view of the first variation example of the second embodiment according to the present invention when the cross-sectional view thereof is viewed in the Z-direction.
FIG. 29 is a plan view of the first variation example of the second embodiment according to the present invention, in which the diffusion member is mounted in an inclined position, when the cross-sectional view thereof is viewed in the Z direction.
FIG. 30 is a graph indicating the simulation results of the relative radiation intensity with respect to the emission angle of ultraviolet light of the first variation example of the second embodiment according to the present invention.
FIG. 31 is a graph indicating the simulation results of the relative radiation intensity with respect to the emission angle of ultraviolet light of the first variation example of the second embodiment according to the present invention when the diffusion member thereof is mounted in an inclined position.
FIG. 32 is a plan view of the second variation example of the second embodiment according to the present invention when the cross-sectional view thereof is viewed in the Z-direction.
FIG. 33 is a graph indicating the simulation results of the relative radiation intensity with respect to the emission angle of ultraviolet light and visible light of the second variation example of the second embodiment according to the present invention.
FIG. 34 is a conceptual view illustrating an example in which a cylindrical lens is used as an optical control member.
FIG. 35 is a conceptual view illustrating a variation example of the second embodiment according to the present invention.
FIG. 36 is a conceptual view illustrating another variation example of the second embodiment according to the present invention.

### DESCRIPTION OF EMBODIMENTS

Hereinafter, an inactivation device for bacteria and/or viruses according to the present invention will be described with reference to the drawings. Note that the following drawings are schematically illustrated, and dimensional ratios and numbers on the drawings do not always match with the actual dimensional ratios and numbers.

The inactivation device for bacteria and/or viruses of the present invention includes an ultraviolet light source and uses the ultraviolet light emitted from the ultraviolet light source to inactivate the bacteria and/or viruses present in a space or onto the surface of an object. In particular, the ultraviolet light source provided in this inactivation device uses an ultraviolet light source emitting ultraviolet light having a main emission wavelength belonging to within a wavelength range from 190 nm or more to less than 240 nm. Ultraviolet light having a wavelength of longer than 240 nm (in particular, deep ultraviolet light (UVC)) is likely to penetrate human skin to the inside of the skin. Hence, cells inside the skin are prone to be damaged. In contrast, ultraviolet light having a wavelength of less than 240 nm is likely to be absorbed by the surface of human skin (e.g., stratum corneum), and is less likely to penetrate human skin to the inside of the skin when the wavelength is shorter, thereby providing less adverse effects of ultraviolet light on human bodies. This is suggested by the fact that the absorption spectrum of proteins constituting skin exhibits a high absorptance for ultraviolet light having a wavelength of less than 240 nm. In particular, ultraviolet light having a shorter wavelength band can provide more safety to human bodies. Hence, the ultraviolet light emitted from the ultraviolet light source provided in the device preferably have a main emission wavelength band belonging to a wavelength range of 237 nm or less, more preferably have a main emission wavelength band belonging to a wavelength range of 235 nm or less, and further preferably have a main emission wavelength band belonging to a wavelength range of 230 nm or less. In addition, since ultraviolet light of an extremely short wavelength tends to generate ozone when emitted in the air, it is more suitable to use ultraviolet light having an emission wavelength band belonging to a wavelength range of 200 nm or longer from the viewpoint of reducing adverse effects of ozone on human bodies. From this viewpoint, the ultraviolet light source provided with the inactivation device preferably includes a light source emitting ultraviolet light having a main emission wavelength belonging to a wavelength band of 200 nm or more to 235 nm or less. The ultraviolet light source provided in the inactivation device is considered to include an LED element or an ultraviolet light lamp (especially an excimer lamp). Hereinafter, an aspect of the former will be described in a first embodiment and an aspect of the latter will be described in a second embodiment.

### First Embodiment

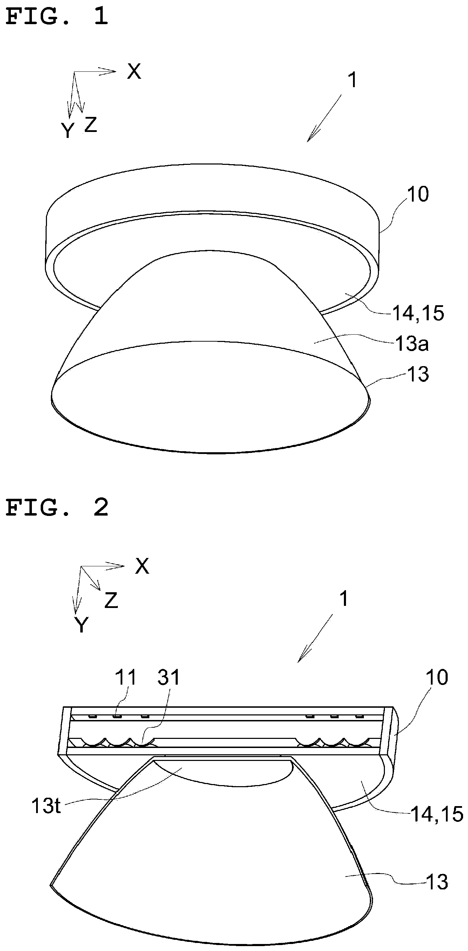

FIG. 1 is a perspective view schematically illustrating a first embodiment of an inactivation device 1. As described below with reference to FIG. 2, the inactivation device 1 includes LED elements 11 as the ultraviolet light source. The ultraviolet light emitted from the LED element 11 is radiated to the outside of the inactivation device 1 through a light-extraction surface 15. FIG. 2 is a perspective view illustrating a cross-section that is cut in a plane perpendicular to the light-extraction surface 15.

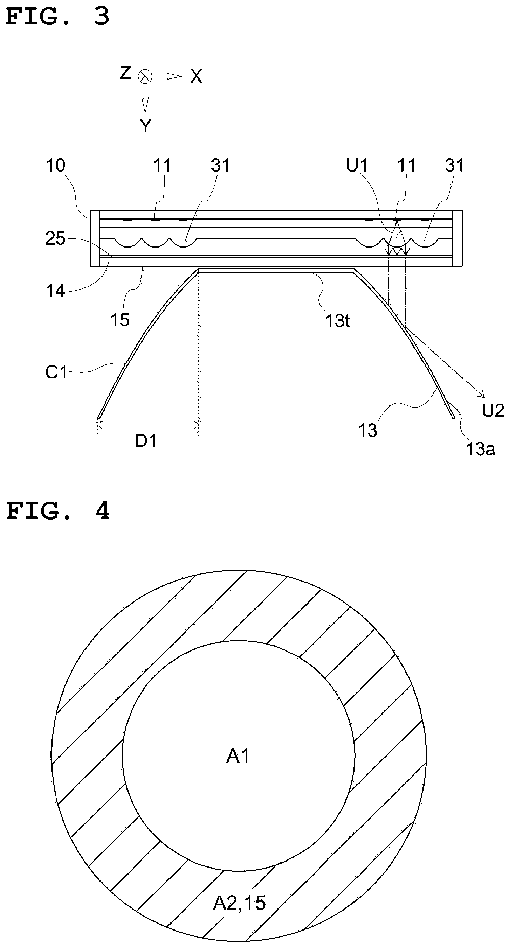

In each of the following figures, the X-Y-Z coordinate system is appropriately given such that the direction parallel to the light-extraction surface is denoted as the X direction, the direction perpendicular to the light-extraction surface is denoted as the Y direction, and the direction orthogonal to the X and Y directions is denoted as the Z direction. Using this definition, FIG. 3 corresponds to a plan view when the cross-sectional view shown in FIG. 2 is viewed in the Z direction. In FIG. 3, a dashed line schematically represents the way in which part of light travels. The same is true in the following drawings.

In the following descriptions, when the direction is expressed with distinguishing a positive direction from a negative one, it is described with a positive or negative sign, such as "+X direction" or "-X direction". When the direction is expressed without distinguishing a positive direction from a negative one, it is simply described as "X direction". In other words, the term "X-direction" simply described in the present specification includes both of the "+X direction" and "-X direction". The same applies to the Y direction and the Z direction.

In the present embodiment, the X direction corresponds to the "first direction" and the Y direction corresponds to the "second direction".

As shown in FIGS. 1 to 3, the inactivation device 1 of the present embodiment includes an enclosure 10, and the plurality of LED elements 11 as the above-mentioned ultraviolet light sources arranged inside this enclosure 10 in the X-Z plane. The LED elements 11 have a main emission wavelength belonging to within a wavelength range from 190 nm or more to less than 240 nm. Examples of the peak wavelength of the LED elements 11 include 235 nm or 226 nm.

In the present embodiment, the plurality of LED elements 11 is annularly arranged. This point will be discussed in detail with reference to FIG. 4.

As shown in FIGS. 1 and 2, the enclosure 10 in the present embodiment has a disk shape, and part of its surface constitutes the light-extraction surface 15. The light-extraction surface 15 is parallel to the X-Z plane. A part of the light-extraction surface 15 is formed with a window member 14 that is made of a material transmitting ultraviolet light U1 emitted from the LED elements 11. The ultraviolet light U1 emitted from the LED elements 11 travels in the +Y direction toward the window member 14. The ultraviolet light U1 passes through the window member 14, then is emitted outside the enclosure 10 through the light-extraction surface 15.

The inactivation device 1 of the present embodiment includes an optical filter 25 attached to this window member 14. This optical filter 25 may be mounted on the light-extraction surface 15, or a surface of the window member 14 facing the LED elements 11. The optical filter 25 serves to transmit ultraviolet light emitted from the ultraviolet light source and that belongs to within a wavelength range from 190 nm or more to less than 240 nm, and suppress the transmission of ultraviolet light having a wavelength range from 240 nm or more to less than 300 nm. The optical filter 25 is formed with, for example, a multilayer film that is constituted by multiple dielectric layers having different refractive indices, and that is designed to exhibit such a wavelength selectivity.

In addition, the inactivation device 1 of the present embodiment houses a collimator lens 31 inside the enclosure 10. The collimator lens 31 has a lens surface at a position corresponding to each LED element 11, reducing the divergence angle of light flux emitted from each LED element 11. Typically, the collimator lens 31 converts the light flux emitted from each LED element 11 into collimated light. In other words, the ultraviolet light U1 emitted from the LED elements 11 is collimated by the collimator lens 31 in the Y direction, then is incident on the optical filter 25 at a small incident angle. After that, the ultraviolet light U2, which has passed through the optical filter 25 and has a wavelength range from 190 nm or more to less than 240 nm, is extracted to the outside of the enclosure 10. For simplification of the figure, the ultraviolet light U1 and the ultraviolet light U2 are shown omitted as appropriate.

As described above, the LED element 11 has a main emission wavelength belonging to within a wavelength range from 190 nm or more to less than 240 nm. However, the emission spectrum of the LED element 11 may include ultraviolet light belonging to within a wavelength band of 240 nm or more to less than 300 nm. Moreover, the emission spectrum thereof may exhibit light intensity at a wavelength band away from the main emission wavelength, i.e., at the tail of the emission spectrum, although the light intensity is very weak compared with that at the peak wavelength (peak intensity). The optical filter 25 is mounted for the purpose of suppressing the traveling of light having a wavelength band of 240 nm or more to less than 300 nm.

The optical filter 25 has a property (incident angle dependency) in which its transmittance and reflectance of each wavelength vary depending on the incident angle of the ultraviolet light U1 incident thereon. In more detail, increasing the incident angle with respect to the optical filter 25 decreases the transmittance in a wavelength range from 190 nm or more to less than 240 nm, and increases the reflectance. This may pose a concern of decreasing the extraction efficiency of ultraviolet light to the outside of the inactivation device 1. For addressing this concern, providing the collimator lens 31 between the LED element 11 and the optical filter 25 makes the ultraviolet light U1 emitted from the LED element 11 be incident onto the optical filter 25 with smaller incident angles, thereby less likely to decrease the light extraction efficiency.

Furthermore, the inactivation device 1 is provided with a reflective member 13 on the +Y side of the light-extraction surface 15. The reflective member 13 includes a reflective surface 13a made of a material that is reflective to the ultraviolet light U2 emitted from the light-extraction surface 15. In the present embodiment, the reflective surface 13a of the reflective member 13 corresponds to an outer surface of a rotator shape, the outer surface being made by rotating a curve C1 (refers to FIG. 3) around the Y direction as its rotation axis. In detail, the reflective member 13 includes an upper surface 13t that is parallel to the light-extraction surface 15 and that has the same area as a first area A1 described below, and an outer side face gently curved from the outer edge of the upper surface 13t. This outer side face corresponds to the reflective surface 13a. As shown in FIG. 3, the width D1 of the reflective surface 13a in the X direction is approximately the same as the width of a second area A2.

When the reflective member 13 has the reflective surface 13a with the shape described above, the ultraviolet light U2 incident on the reflective surface 13a through the light-extraction surface 15 is reflected to be directly away from the enclosure 10 in the direction of the X-Z plane (only the X direction is illustrated in FIG. 3). Examples of materials that can be used for the reflective surface 13a include bright aluminum, aluminum deposited on the reflective member, and a dielectric multilayer film formed on the reflective member. The reflective member 13 can be made of these materials, or these materials can be used only on the reflective surface 13a.

FIG. 4 is a schematic view illustrating an arrangement of the plurality of LED elements 11 when viewed from the Y direction. In FIG. 4, the area in which the LED elements 11 are arranged is hatched. In other words, the inactivation device 1 of the present invention, when viewed from the Y direction, has the first area A1 in which no LED element 11 is arranged and a second area A2 in which the plurality of LED elements 11 are arranged in a manner that the LED elements surround the first area A1. The light-extraction surface 15 shows a shape corresponding to the second area A2. In other words, as shown in FIG. 4, the light-extraction surface 15 has an annular shape when viewed from the Y direction.

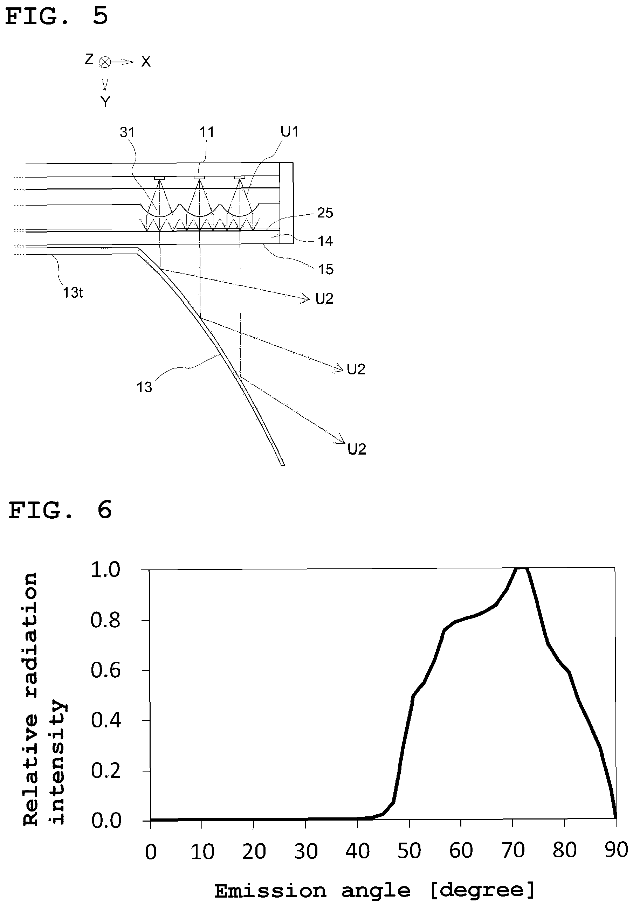

FIG. 5 is an enlarged view illustrating in detail the traveling directions of the ultraviolet light U2, which is partially omitted in FIG. 3. As shown in FIG. 5, the traveling direction of the ultraviolet light U2 that has passed through the light-extraction surface 15 is changed by the reflective member 13, increasing the emission angle of the ultraviolet light U2. In other words, this configuration preferentially enables the ultraviolet light irradiation in the wide-angle direction, which has a large emission angle with respect to the light-extraction surface 15, thereby increasing the illuminance in an area facing in the wide-angle direction.

FIG. 6 is a graph indicating the results of a simulation S1 in which the relative radiation intensity of ultraviolet light U2 emitted from the inactivation device 1 was calculated with respect to the emission angle thereof.

### Simulation S1

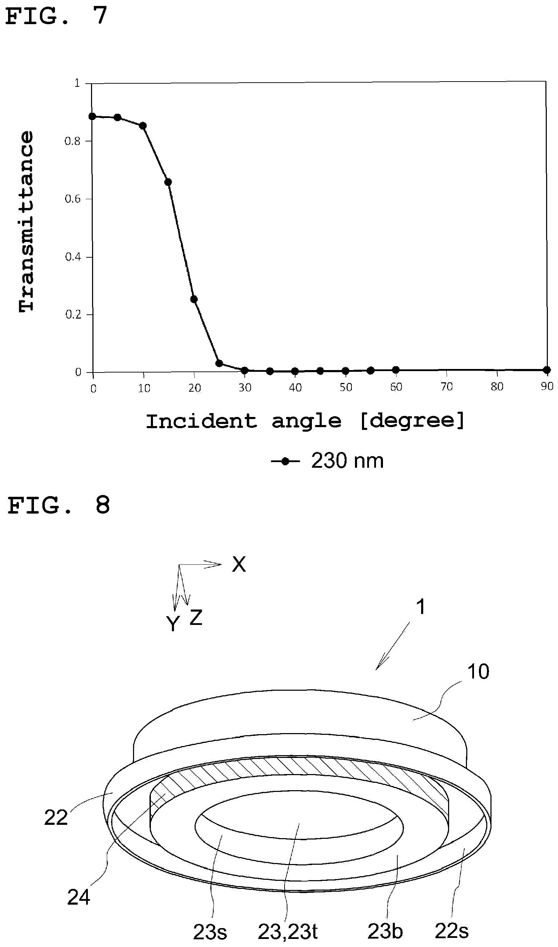

Simulation S1 was performed with considering the configuration of the first embodiment shown in FIGS. 1 to 3. In Simulation S1, the LED elements 11 were concentrically arranged in three rows with equal spacing in a circumferential direction. Specifically, 20 units of the surface-emitting LED elements of 0.8 mm square were mounted on a circle of radius 10 mm, 27 units thereof on a circle with a radius of 13 mm, and 33 units thereof on a circle with a radius of 16 mm. The shape of the reflective surface 13a of the reflective member 13 was configured to be formed by rotating a cubic spline curve around the center of the circle in which the LED elements 11 were mounted. When viewed in the axial direction (hereinafter referred to as "normal direction" for convenience), the reflective surface 13a was configured to extend outside the position of the LED elements 11 disposed at the outermost row. In addition, the collimator lens 31 was set to make ultraviolet light having an incident angle of 37 degrees or less at the incident surface of the collimator lens 31 substantially parallel in the normal direction. The simulation was performed with considering that the optical filter 25 was set to have characteristics shown in FIG. 7.

The relative radiation intensity is determined by dividing the radiation intensity obtained from the illuminance measurement at any given emission angle with the maximum radiation intensity. In practice, referring to JIS C 8105-5 "Light Distribution Measurement Methods" and using an illuminance meter sensitive to the peak wavelength of the ultraviolet light source, the relative radiation intensity is determined by dividing the radiation intensity obtained from the illuminance measurement at each emission angle with the maximum value of radiation intensity at all emission angles. The emission angle is defined to set the center of the light-extraction surface 15 as the origin, and the Y direction as 0 degrees, as described above. The direction with an emission angle of 90 degrees corresponds to the direction parallel to the X-Z plane; and note that the direction with an emission angle of 90 degrees can be considered in the direction of 360 degrees, including the X direction and the Z direction. Hereinafter, in the present specification, the graphs of relative radiation intensity with respect to the emission angle represent the average value for all directions of 360 degrees.

The traveling direction of the ultraviolet light U2 is changed by the reflective member 13 (see FIG. 5), the effect of which will be described with reference to FIG. 6. As shown in FIG. 6, the relative radiation intensity of ultraviolet light indicates 0 when the emission angle is in a range from 0 degrees or more to less than 40 degrees, and the value of the relative radiation intensity of ultraviolet light rises when the emission angle exceeds 40 degrees. The relative radiation intensity of ultraviolet light gradually increases as the emission angle increases, reaches the maximum value at around 70 degrees, and then gradually decreases to 0 at approximately 90 degrees (see FIG. 6). In other words, the reflective member 13 changes the emission angle of the ultraviolet light U2 to a wide-angle direction, thus resulting in the irradiation of ultraviolet light in the range from 40 degrees to 90 degrees. Hence, the illuminance in the area irradiated with ultraviolet light in the wide-angle direction can be higher than the illuminance in the area irradiated with ultraviolet light in the 0 degrees direction.

As mentioned above, when the radiation intensity of the ultraviolet light U2 emitted from the light-extraction surface 15 of the inactivation device 1 is broken down with angle, the angle that indicates the maximum value (peak angle) is preferably designed to be in the range from 40 degrees or more to less than 90 degrees, more preferably in the range from 40 degrees or more to less than 85 degrees, and even more preferably in the range from 40 degrees or more to less than 80 degrees. In the present embodiment, the peak angle is located around 70 degrees.

Although the results of the present simulation S1 are calculated under the above conditions, it is understood that the configuration according to the present invention makes the radiation intensity in the wide-angle direction higher than that in the 0 degrees direction, even if the conditions such as the size ratio and the number of LED elements differ from the above conditions. Hence, the inactivation device for bacteria and/or viruses according to the present invention enables the ultraviolet light irradiation to a wider area compared with the case in which the illuminance in an area irradiated with ultraviolet light in the 0 degrees direction is maximized, when the ultraviolet light irradiation dose is controlled in accordance with the ACGIH standard.

### Variation example

Hereinafter, the variation examples of the first embodiment will be described with reference to the drawings.

### First variation example

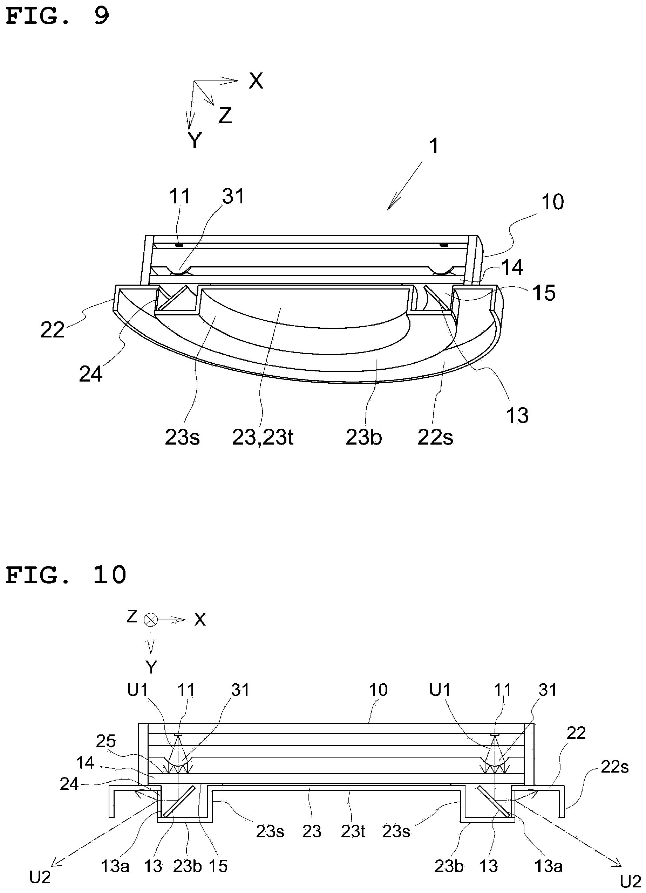

FIG. 8 is a perspective view schematically illustrating a first variation example of the first embodiment of the inactivation device 1. FIG. 9 is a perspective view illustrating a cross-section cut in a plane perpendicular to the light-extraction surface 15. FIG. 10 corresponds to a plan view when the cross-sectional view shown in FIG. 9 is viewed in the Z direction. The following is a description of the configuration of the first variation example, focusing on the points that differ from those of the first embodiment.

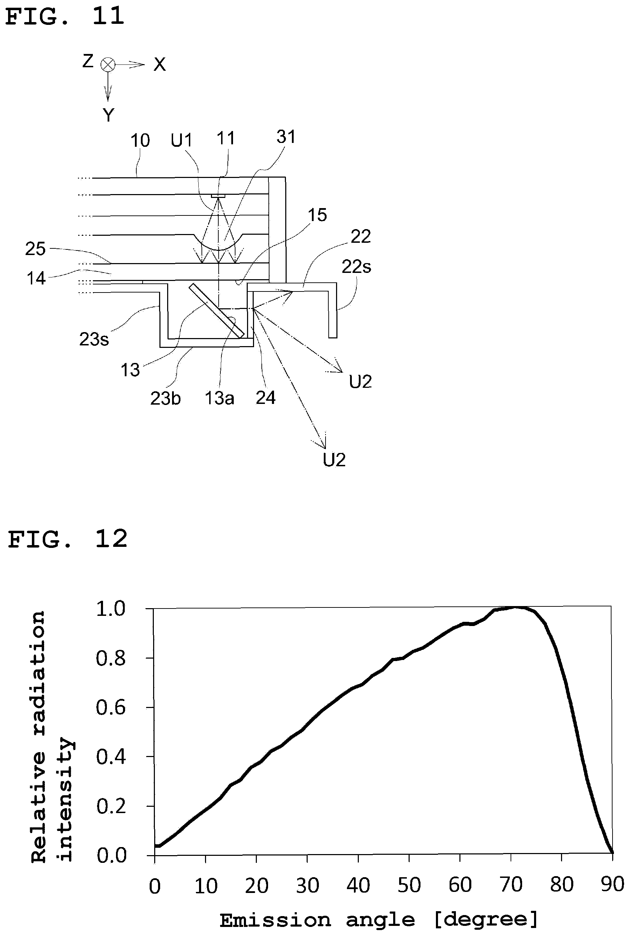

As shown in FIGS. 8 to 10, the inactivation device 1 of the first variation example is provided with the reflective member 13 on the +Y side of the light-extraction surface 15. The discussion similar to that for the first embodiment can be made in that the reflecting member 13 reflects the ultraviolet light U2 emitted from the light-extraction surface 15 directly away from the enclosure 10 with respect to the X-Z plane direction (only the X direction is illustrated in Fig. 10). In the first variation example of the inactivation device 1, the reflective member 13 has a conical trapezoidal shape showing a convex shape with respect to the -Y direction and is disposed in the space enclosed by a side portion 23s and a bottom portion 23b of a second light-shielding member 23, which will be described below, and the light-extraction surface 15 in an area facing the second area A2 (see FIG. 4) in the Y direction. In addition, the reflective member 13 is disposed to surround the first area A1 (see FIG. 4) when viewed from the Y direction. FIG. 11 is an enlarged view of an area near the reflective member 13 in FIG. 10. As shown in FIG. 11, the reflective surface 13a is provided on the outer side face of the reflective member 13. As shown in FIGS. 10 and 11, the reflective surface 13a is inclined to the light-extraction surface 15, which is parallel to the X-Z plane, and reflects the ultraviolet light U2 incident thereon to the outside of the enclosure 10.

In addition, as shown in FIGS. 8 to 10, the inactivation device 1 of the first variation example is provided with a first light-shielding member 22 that blocks, of the ultraviolet light U2 that has been reflected by the reflective surface 13a, the traveling of the ultraviolet light U2 that travels in the -Y direction, in other words, closer toward a location in which the LED elements 11 are mounted. This will be described in detail with reference to FIG. 11.

As shown in FIG. 11, of the ultraviolet light U2 emitted from the light-extraction surface 15, reflected on the reflective surface 13a, and then diffused in a diffusion member 24, which will be described later, the first light-shielding member 22 blocks the traveling of the ultraviolet light U2 traveling toward the LED elements 11 in the -Y direction. In this way, providing the first light-shielding member 22, which blocks the ultraviolet light U2 traveling toward the side where the LED elements 11 are mounted in the inactivation device 1, in other words, the opposite of an area where the ultraviolet light irradiation is intended, prevents irradiation to an area where ultraviolet light irradiation is undesired.

In the inactivation device 1 of the first variation example, the first light-shielding member 22 has a key shape with a constant width in the X direction and a vertical bend in the +Y direction. The first light-shielding member 22 has a rotation body that is made by rotating this key-shape around a rotation axis in the +Y direction. In other words, the first light-shielding member 22 is an annular plate member with a side wall 22s on its outer periphery, and is connected to the end of the enclosure 10 in the +Y direction. The width of the side wall 22s in the Y-direction may be equal to or greater than the width of the diffusion member 24 in the Y-direction. In other words, the maximum position of the side wall 22s in the Y direction may be equal to or greater than the maximum position of the diffusion member 24 in the Y direction. In addition, the width of the side wall 22s in the Y direction may be equal to or greater than the width of the reflective member 13 in the Y-direction. Furthermore, the end of the side wall 22s in the +Y direction may be located at the same position, or toward the +Y direction, of the end of the diffusion member 24 in the +Y direction.

The first light-shielding member 22 may be a flat plate with its principal plane in the X-Z plane, or, as described above, the first light-shielding member 22 may have the side wall 22s extending toward the emission direction (+Y direction in FIG. 11) with respect to the light-extraction surface 15. The first light-shielding member 22 may also be formed such that the side wall 22s is partially provided. Having such a shape of the side wall 22s enables the ultraviolet light U2 traveling the opposite of an area where the ultraviolet light irradiation is intended to be more effectively blocked. In addition, the side wall 22s provides a suitable configuration in that adjusting the width of the side wall 22s in the Y-direction can adjust the emission direction of ultraviolet light U2. In the first light-shielding member 22, the part having a flat plate shape with its main plane in the X-Z plane and the part having the shape shown in the side wall 22s may be individually designed as separate components.

Examples of the materials that can be used as the first light-shielding member 22 include black anodized aluminum, glass that fails to transmit ultraviolet light having a wavelength less than 300 nm such as soda-lime glass, electroless nickel plated on components, and ultraviolet light absorbers coated on components. The first light-shielding member 22 can be made of these materials, or these materials can be used only on the surface on which ultraviolet light U2 is incident. The first light-shielding member 22 may also be a component that absorbs the ultraviolet light U2 provided that it blocks the traveling of the ultraviolet light U2.

Furthermore, the inactivation device 1 of the first variation example is provided with a second light-shielding member 23, as shown in FIGS. 8 to 10. The second light-shielding member 23 blocks the traveling of at least part of the ultraviolet light emitted from the light-extraction surface 15 in the +Y direction. For example, it blocks the traveling of ultraviolet light emitted from the first area A1 (see FIG. 4). The second light-shielding member 23 can be made of any material as long as it serves to block the traveling of ultraviolet light. As an example, the second light-shielding member 23 can be formed of the same or similar material as the first light-shielding member 22. This configuration, in addition to the low arrangement density of the LED elements 11 in the first area A1, enables the illuminance in an area facing in the 0 degrees direction to be relatively lower than the illuminance in the surrounding region.

In the inactivation device 1 of the first variation example, the second light-shielding member 23 is in contact with the light-extraction surface 15 on the +Y side, and has an upper surface 23t parallel to the light-extraction surface 15. This upper surface 23t of the second light-shielding member 23 may have approximately the same area as the first area A1. Furthermore, the second light-shielding member 23 has a side portion 23s extending from the outer edge of the upper surface 23t in the Y direction and a bottom portion 23b extending from this side portion 23s further away from the upper surface 23t. In other words, the second light-shielding member 23 has a so-called silk hat shape with a flat upper surface 23t and a cylindrical side face with a flange. The reflective member 13 is disposed in the space enclosed between the side portion 23s and the bottom portion 23b. The upper surface of the second light-shielding member 23, which is in contact with the light-extraction surface in the +Y direction, blocks the ultraviolet light U2 emitted from the first area A1.

In addition, in the inactivation device 1 of the first variation example, as shown in FIGS. 8 to 11, the diffusion member 24 is disposed between the reflective surface 13a and the first light-shielding member 22 in a manner it surrounds the outside of the reflective member 13. In FIG. 8, the diffusion member 24 is illustrated as a hatched area. With reference to FIG. 11, the traveling direction of the ultraviolet light U2 that has passed through the light-extraction surface 15 is changed by the reflective member 13 and then further diffused by the diffusion member 24. In other words, ultraviolet light is preferentially radiated in a wide-angle direction with a large emission angle with respect to the light-extraction surface 15, and moreover, the ultraviolet light U2 is diffused through the diffusion member 24, hence it is less likely that an emission direction with a significantly high radiation intensity will appear with respect to the emission direction of the ultraviolet light U2 emitted from the inactivation device 1. Hence, this configuration is capable of further relaxing the limitations on the output power and irradiation time of the ultraviolet light source required to comply with the standard of irradiation dose specified by the ACGIH. In the inactivation device 1 of the first variation example, the diffusion member 24 is disposed such that it connects the first light-shielding member 22 to the second light-shielding member 23; however, it may be fixed to either of them. Examples of the diffusion member 24 include a sheet of fluorine resin sheet such as PTFE.

FIG. 12 is a graph indicating the results of a simulation S2 in which the relative radiation intensity of ultraviolet light U2 emitted from the inactivation device 1 of the first variation example was calculated with respect to the emission angle thereof.

### Simulation S2

The simulation S2 was performed with considering the configuration of the first variation example of the first embodiment shown in FIGS. 8 to 10. In the simulation S2, the LED elements 11 were annularly arranged in one row. Specifically, 41 units of the surface-emitting LED elements of 0.8 mm square were arranged in the circumference of a circle with a radius of 2.0 mm. The reflective surface 13a of the reflective member 13 had a conical trapezoidal shape, and the reflective surface 13a is inclined at 45 degrees with respect to the light-extraction surface 15. Furthermore, the diffusion member 24 was arranged to surround the outside of the reflective surface 13a (see FIG. 9). Other conditions are the same as those in the simulation S1.

With reference to FIG. 12, described will be the effect of the traveling direction of the ultraviolet light U2 is changed by the reflective member 13, and diffused by the diffusion member 24 (see FIG. 11). As shown in FIG. 12, in the inactivation device 1 of the first variation example, the relative radiation intensity of ultraviolet light is less than 0.1 at an emission angle of 0 degrees, gradually increases as the emission angle increases, and reaches the maximum at around 70 degrees. In other words, the illuminance in an area irradiated with ultraviolet light in the wide-angle direction is higher than the illuminance in an area irradiated with ultraviolet light in the 0 degrees direction. Furthermore, when FIG. 6, which corresponds to the results of simulation S1 based on the inactivation device 1 without the diffusion member 24, is compared with FIG. 12, which corresponds to the results of simulation S2 based on the inactivation device 1 of the second variation example with the diffusion member 24, it is understood that providing the diffusion member 24 in the inactivation device 1 decreases the difference in radiation intensity at each emission angle. Therefore, when the inactivation device 1 is used to radiate ultraviolet light, an emission direction with a significantly high radiation intensity is less likely to appear, effectively performing the ultraviolet light irradiation in the space or onto the surface of an object.

Although the results of the present simulation S1 are calculated under the above conditions, it is understood that the configuration according to the present invention makes the radiation intensity of ultraviolet light in the wide-angle direction higher than that in the 0 degrees direction, even if the conditions such as the size ratio and the number of LED elements differs from the above conditions.

### Second variation example

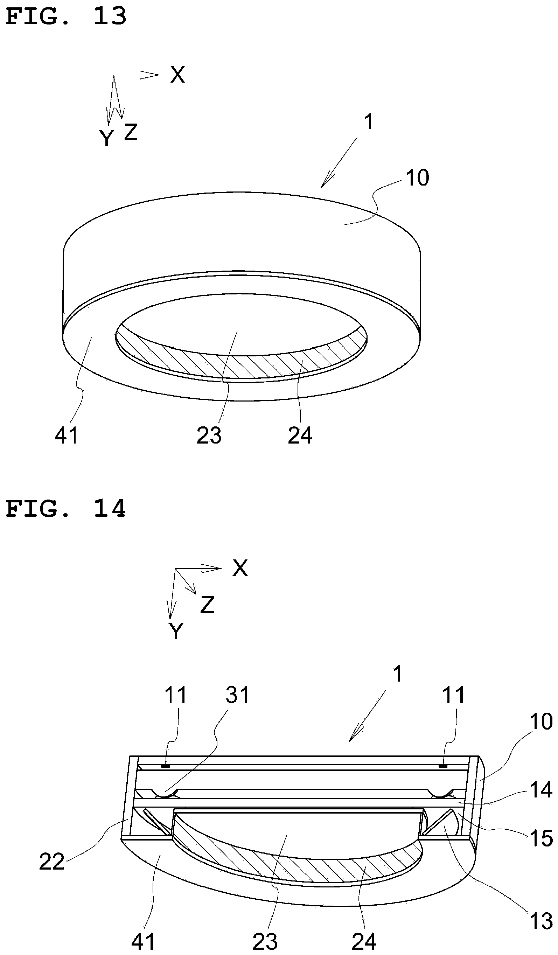

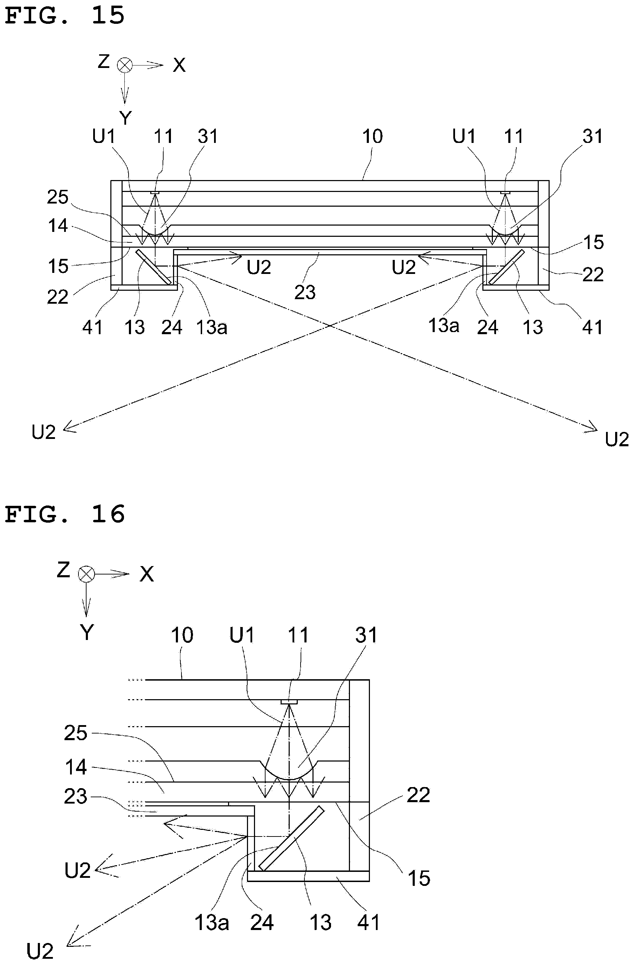

FIG. 13 is a perspective view schematically illustrating a second variation example of the first embodiment of the inactivation device 1, and FIG. 14 is a perspective view illustrating a cross-section cut in a plane perpendicular to the light-extraction surface 15. FIG. 15 corresponds to a plan view when the cross-sectional view shown in FIG. 14 is viewed in the Z direction. The following is a description of the configuration of the second variation example, focusing on the points where it differs from the first embodiment.

As shown in FIGS. 13 to 15, the inactivation device 1 of the second variation example is provided with the reflective member 13 on the +Y side of the light-extraction surface 15. The reflective member 13 includes the reflective surface 13a that makes the traveling direction of the ultraviolet light U2 emitted from the light-extraction surface 15 change the direction away from the enclosure 10 after traveling toward the center area in the X direction of the enclosure 10. In the inactivation device 1 of the second variation example, the reflective member 13 has a conical trapezoidal shape showing a convex shape with respect to the +Y direction, and is disposed on a mounting member 41 described below in an area facing the second area A2. The reflective member 13 is arranged to surround the first area A1 (FIG. 4) when viewed from the +Y direction. FIG. 16 is an enlarged view illustrating an area near the reflective member 13 in FIG. 15. As shown in FIG. 16, the reflective surface 13a is provided on the inner surface of the reflective member 13. The reflective surface 13a is inclined with respect to the light-extraction surface 15, thus reflecting the ultraviolet light U2 incident thereon toward the inside of the enclosure 10, more particularly in the direction traveling toward the center area in the X direction.

In the inactivation device 1 of the second variation example, as shown in FIGS. 13 to 15, the first light-shielding member 22 is provided at the end of the side face of the enclosure 10 in the +Y direction. The first light-shielding member 22 is configured to be a cylinder having the same width as the enclosure 10. As mentioned above, in the inactivation device 1 of the second variation example, the traveling direction of the ultraviolet light U2 incident on the reflective surface 13a of the reflective member 13 is changed to approach mainly toward the inside of the enclosure 10, but part of the ultraviolet light U2 may travel to the outside of the inactivation device 1 or to the side where the LED elements 11 are mounted. The first light-shielding member 22 is provided for the purpose of suppressing the irradiation of the ultraviolet light U2 to an area where ultraviolet light irradiation is unintended.

Furthermore, the inactivation device 1 of the second variation example is provided with the second light-shielding member 23, as shown in FIGS. 13 to 15. The second light-shielding member 23 blocks the traveling of the ultraviolet light emitted from the first area A1 (see FIG. 4), thus the same discussions can be made with those for the second light-shielding member 23 in the first variation example.

In addition, in the inactivation device 1 of the second variation example, as shown in FIGS. 13 to 16, the diffusion member 24 is disposed at an inner position of the reflective surface 13a and on the outer edge of the second light-shielding member 23. In other words, the reflective surface 13a is disposed to surround the outside of the diffusion member 24. In FIGS. 13 and 14, the diffusion member 24 is illustrated as a hatched area. As shown in FIGS. 15 and 16, the ultraviolet light U2 reflected at the reflective surface 13a is diffused by the diffusion member 24, and is extracted from the inactivation device 1 into the space. In the inactivation device 1 of the second variation example, the diffusion member 24 is disposed to connects the second light-shielding member 23 to the mounting member 41 described below; however, it may be fixed to either of them.

The inactivation device 1 of the second variation example has the annular mounting member 41 having the similar area as the second area A2 (see FIG. 4). This mounting member 41 is disposed to connect the first light-shielding member 22 to the diffusion member 24. The mounting member 41 may be made of the same material as the first light-shielding member 22, and may be integrated with the first light-shielding member 22. Since only a fraction of the ultraviolet light U2 incident on the reflective member 13 is considered to travel in the Y direction, the mounting member 41 can be any configuration.

In the configuration described above, the reflective member 13 is disposed to be connected to the mounting member 41; however, it can be fixed to, for example, the first light-shielding member 22 or the enclosure 10.

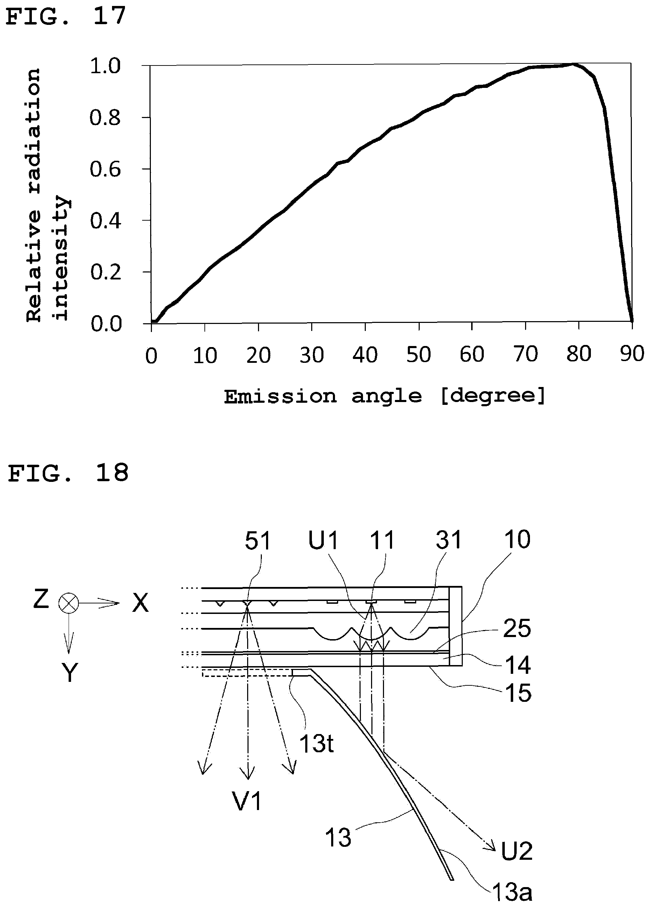

FIG. 17 is a graph indicating the result of a simulation S3 in which the relative radiation intensity of ultraviolet light U2 emitted from the inactivation device 1 of the second variation example was calculated with respect to the emission angle thereof.

### Simulation S3

The simulation S3 was performed with considering the configuration of the second variation example of the first embodiment shown in FIGS. 13 to 15. The simulation S3 has a condition different from that of the simulation S2 in that the reflective surface 13a of the reflective member 13 surrounds the outside of the diffusion member 24 (see FIG. 14), and the other conditions are the similar to those of the simulation S2.

As shown in FIG. 17, in the inactivation device 1 of the second variation example, the relative radiation intensity of ultraviolet light is less than 0.1 at an emission angle of 0 degrees and gradually increases as the emission angle increases, and reaches a maximum radiation intensity of ultraviolet light at around 80 degrees. In other words, the illuminance in the area irradiated with ultraviolet light in the wide-angle direction is higher than the illuminance in the area irradiated with ultraviolet light in the 0 degrees direction. Furthermore, when FIG. 6, which corresponds to the results of simulation S1 based on the inactivation device 1 without the diffusion member 24, is compared with FIG. 17, which corresponds to the results of simulation S3 based on the inactivation device 1 of the second variation example with the diffusion member 24, it is understood that providing the diffusion member 24 in the inactivation device decreases the difference in radiation intensity at each emission angle. Therefore, when the inactivation device 1 is used to radiate ultraviolet light, an emission direction with a significantly high radiation intensity is less likely to appear, effectively performing the ultraviolet light irradiation in the space or onto the surface of an object.

Although the results of the present simulation S3 are calculated under the above conditions, it is understood that the configuration according to the present invention makes the radiation intensity of ultraviolet light in the wide-angle direction higher than that in the 0 degrees direction, even if the conditions such as the size ratio and the number of LED elements differs from the above conditions.

In the inactivation device 1 of the second variation example, as described above, the reflective surface 13a of the reflective member 13 reflects the ultraviolet light U2 that has passed through the light-extraction surface 15 toward the inside of the enclosure 10. In this way, even when the optical axis of ultraviolet light U2 is changed in the direction away from the enclosure 10 after reflecting toward the inside of the enclosure 10 and traveling toward the center area of the enclosure 10 in the X direction, it is possible to increase the illuminance in the area irradiated with ultraviolet light in the wide-angle direction of the emission angle.

Compared with the inactivation device 1 described above with reference to FIGS. 1 to 5, the inactivation device 1 of the second variation example 1 can reduce the size of the reflective member 13 relative to the enclosure 10. In addition, the inactivation device 1 of the second variation example differs from the inactivation device 1 of the first variation example described above with reference to FIGS. 9 to 11 in that there is no need to provide the first light-shielding member 22 on the outside of the enclosure 10 with respect to the X-Z plane. (In FIG. 15, only the X direction is shown.) Thus, the inactivation device 1 of the second variation example can be designed to be compact in its overall configuration, leading to a suitable configuration for the arrangement in a room.

### Other variation examples

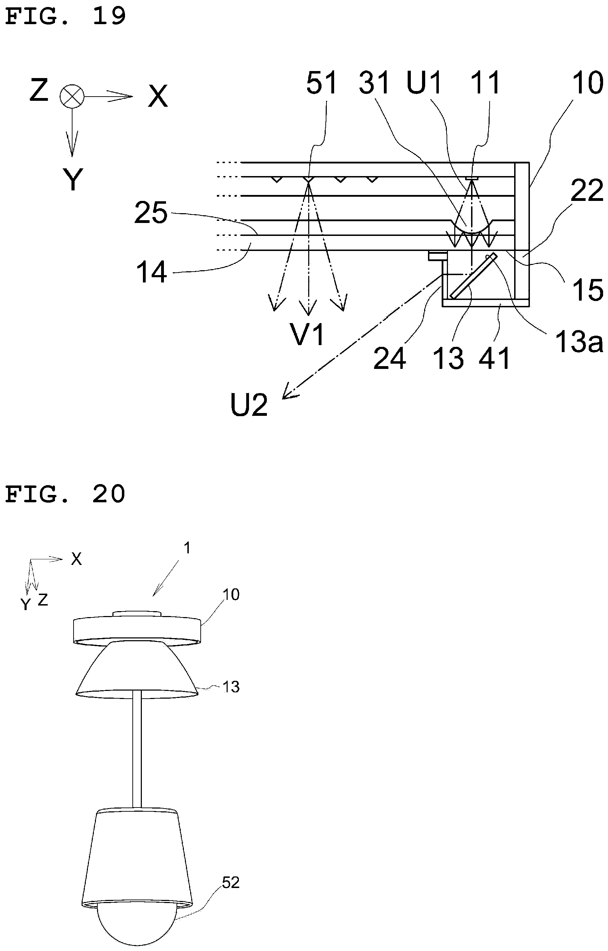

Hereinafter, other variation examples of the inactivation device 1 of the first embodiment will be described.
< 1 >
   The above describes that the collimator lens 31 is housed inside the enclosure 10, and the window member 14 is provided with the optical filter 25. The optical filter 25 is employed to limit the irradiation of ultraviolet light having wavelengths considered harmful to human bodies. However, if such limitation is not necessary, the inactivation device 1 need not necessarily be provided with the optical filter 25. When the optical filter 25 is not provided, the light-extraction surface 15 may be configured to be an opening. Such cases include the case in which the inactivation device 1 performs ultraviolet light irradiation in a place where little or no people come and go, or the case in which the inactivation device 1 performs ultraviolet light irradiation far enough away from a place where people are present. The same holds true for the case in which the inactivation process is performed only during the time of day when no people are present.
<2>
   As mentioned above, the collimator lens 31, which is provided in the inactivation device 1, enables the ultraviolet light U1 emitted from the LED elements 11 to be incident at smaller incident angles with respect to the light-extraction surface 15, thus most of the ultraviolet light U2 that has passed through the light-extraction surface 15 can be guided to the position of the reflective member 13. However, although the ultraviolet light U1 emitted from the LED elements 11 travels with a certain divergence angle, if the LED elements 11 are sufficiently in close to the light-extraction surface 15, most of the ultraviolet light U1 is incident at a sufficiently small angle to the light-extraction surface 15. In this case, the collimator lens 31 need not necessarily be provided.
<3>
   In addition, the collimator lens 31 and the optical filter 25 may also be configured to be integrated with the LED elements 11 as an LED package.
<4>
   Furthermore, the above describes that the LED elements 11 as an ultraviolet light source are not mounted in the first area A1. However, as shown in FIGS. 18 and 19, visible light sources 51 may be mounted in the first area A1 in the enclosure 10, and the visible light emitted from the visible light source 51 may be used for illumination. FIGS. 18 and 19 schematically illustrate the manner in which part of visible light travels with double-dashed lines. The same is true in the following drawings.

FIG. 18 is a cross-sectional view of an example in which the visible light sources 51 are mounted in the first area A1 of the first embodiment. In this case, the area of the upper surface 13t of the reflective member 13 may be reduced.

In addition, FIG. 19 illustrates a cross-sectional view of an example in which the visible light source 51 is mounted in the first area A1 of the second variation example of the first embodiment (see FIGS. 14 to 16). The structure shown in FIG. 19 is not provided with the second light-shielding member 23 compared with that in FIG. 16. Also another example includes that, while the second light-shielding member 23 is provided as similar to the example in FIG. 16, the second light-shielding member 23 may be made of a material that fails to transmit ultraviolet light but transmits visible light.

The configuration shown in FIG. 18 or FIG. 19 enables visible light V1 emitted from the visible light source 51 mounted in the first area A1 to be extracted from the inactivation device 1, thus the first area A1 can be used as an area for illumination while ultraviolet light irradiation is performed via the second area A2. Since the optical filter 25 has little effect on the above-mentioned incident angle dependency in the wavelength range of the visible light V1, thus the visible light V1 can be extracted outside the inactivation device 1 even when the collimator lens 31 is not configured at the position facing the visible light source 51, as shown in FIGS. 18 and 19.
<5>
   A visible light illumination 52 can also be mounted at a distance away from reflective member 13 on the +Y side. FIG. 20 is a perspective view illustrating an example in which the visible light illumination 52 is mounted on the +Y side of the reflective member 13 of the first embodiment and the first area A1 is used for an area for illumination. In this case, the +Y direction is typically oriented vertically downward. In the first variation example and the second variation example, the visible light illumination 52 may be mounted on the opposite side of the LED elements 11 with respect to the second light-shielding member 23.
<6>
   Furthermore, in the second variation example, a phosphor layer that absorbs ultraviolet light and emits visible light may be formed on the opposite side of the LED elements 11 with respect to the second light-shielding member 23. In the case of forming the phosphor layer, the front surface of the phosphor layer may be coated with a fluoropolymer or the like that transmits ultraviolet light and visible light, or a fluoropolymer film containing phosphor may be attached in advance in order to prevent the scattering of the phosphor layer.

### Second Embodiment

Hereinafter, the second embodiment will be described with reference to the drawings. Elements in common with the first embodiment are assigned to the same symbols, and their descriptions are omitted as appropriate.

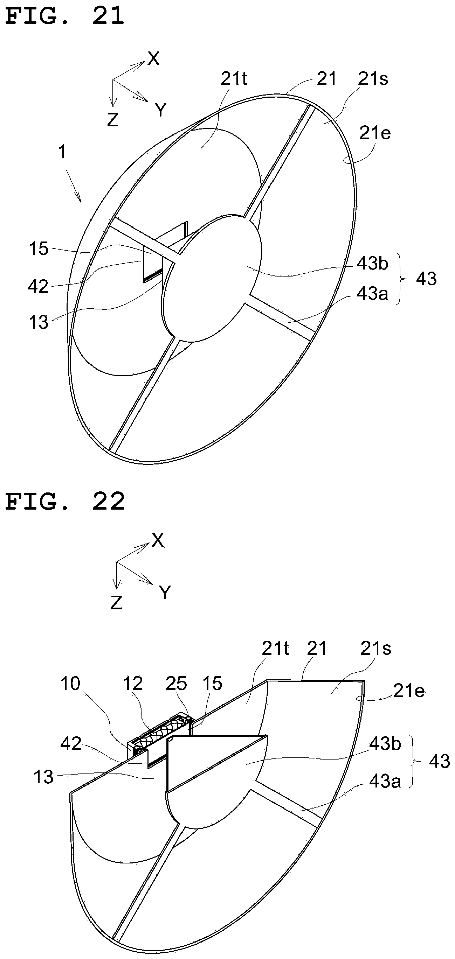

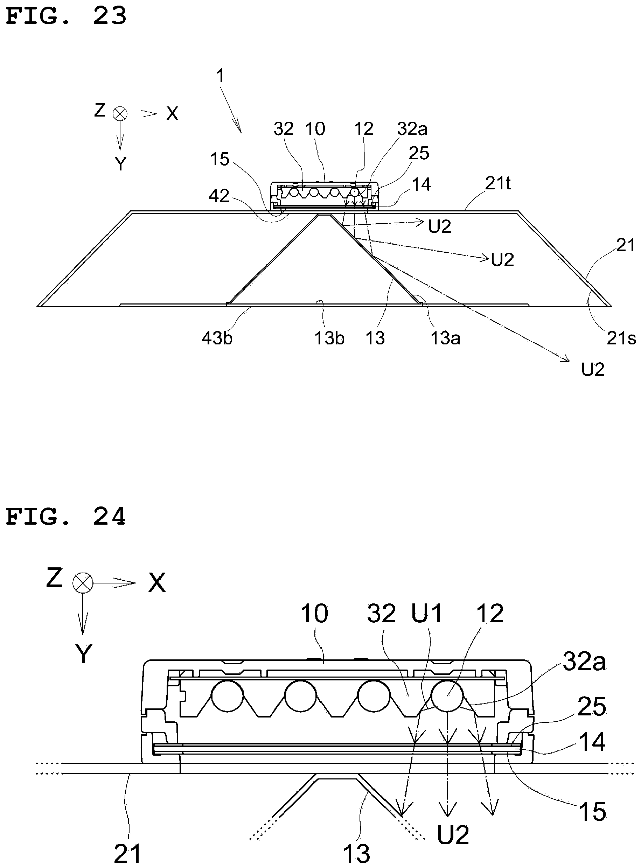

FIG. 21 is a perspective view schematically illustrating the second embodiment of the inactivation device 1. As described below with reference to FIG. 24, the inactivation device 1 includes excimer lamps 12 as an ultraviolet light source. The ultraviolet light emitted from the excimer lamps 12 is radiated to the outside of the inactivation device 1 through the light-extraction surface 15. FIG. 22 is a perspective view illustrating a cross-section cut in a plane perpendicular to the light-extraction surface 15. In addition, FIG. 23 corresponds to a plan view when the cross-sectional view shown in FIG. 22 is viewed in the Z direction.

As shown in FIGS. 21 to 23, the inactivation device 1 of the present embodiment is provided with the enclosure 10, and the above-mentioned excimer lamps 12 as an ultraviolet light source is mounted inside the enclosure 10. The excimer lamp 12 emits ultraviolet light having a main emission wavelength belonging to within a wavelength range from 190 nm or more to less than 240 nm. In other words, the luminescent gas sealed in the excimer lamp 12 consists of a material that emits ultraviolet light U1 having a main emission wavelength of 190 nm or more to less than 240 nm during the excimer light emission. Examples of the luminescent gas include KrCl, KrBr, and ArF.

For example, when the luminescent gas contains KrCl, the excimer lamp 12 emits ultraviolet light U1 having a main peak wavelength of near 222 nm. When the luminescent gas contains KrBr, the excimer lamp 12 emits ultraviolet light U1 having a main peak wavelength of near 207 nm. When the luminescent gas contains ArF, the excimer lamp 12 emits ultraviolet light U1 having a main peak wavelength of near 193 nm.

In the present embodiment, the case in which the four excimer lamps 12 are housed in the enclosure 10 will be described as an example (see FIG. 23); however, the number of excimer lamps 12 can be one, two, three, or five or more.

As shown in FIGS. 22 and 23, in the present embodiment, the enclosure 10 approximately has a rectangular parallelepiped shape, and part of its surfaces constitutes the light-extraction surface 15. The light-extraction surface 15 is parallel to the X-Z plane. Part of the light-extraction surface 15 is formed by the window member 14 made of a material that transmits ultraviolet light U1 emitted from the excimer lamp 12. The ultraviolet light U1 emitted from the excimer lamp 12 travels in the +Y direction toward the window member 14. Then, the ultraviolet light U1 passes through the window member 14 and emits to the outside of the enclosure 10 through the light-extraction surface 15.

The inactivation device 1 of the present embodiment is provided with the optical filter 25 that is attached to the window member 14, which is the similar to the first embodiment.

Furthermore, the inactivation device 1 of the present embodiment is provided with an electrode block 32 having tapered reflective surfaces 32a and that is disposed between the excimer lamps 12 and the light-extraction surface 15 inside the enclosure 10. FIG. 24 is an enlarged view illustrating an area near the excimer lamps 12 in FIG. 23. As shown in FIG. 24, the reflective surfaces 32a of the electrode blocks 32 are arranged to correspond to the respective excimer lamps 12, reducing the divergence angle of the light flux emitted from each excimer lamp 12. In other words, the ultraviolet light U1 emitted from the excimer lamps 12 is reflected by the tapered reflective surfaces 32a of the electrode block 32 and incident on the optical filter 25 at smaller incident angles. Then, the ultraviolet light U2 that has passed through the light-extraction surface 15 and that has a wavelength of 190 nm or more to less than 240 nm is extracted to the outside of the enclosure 10. Being made of a conductive metal material that exhibits conductivity, the electrode block 32 can serve as an electrode of the excimer lamp 12. For simplification of the figure, the ultraviolet light U1 and the ultraviolet light U2 are shown omitted as appropriate.

As described above, the excimer lamp 12 has a main emission wavelength belonging to within a wavelength range from 190 nm or more to less than 240 nm. However, the emission spectrum of the excimer lamp 12 may exhibit light intensity at a wavelength band away from the main emission wavelength, i.e., at the tail of the emission spectrum, although the light intensity is very weak compared with that at the peak wavelength (peak intensity). The optical filter 25 is mounted for the purpose of suppressing the traveling of light having weak light intensity in a longer wavelength band than the main emission wavelength.

As described above, the optical filter 25 has a property (incident angle dependency) in which the transmittance and reflectance of each wavelength vary depending on the incident angle of the ultraviolet light U1 incident thereon. For addressing this property, providing the electrode block 32 having the tapered reflective surfaces 32a between the excimer lamp 12 and the optical filter 25 makes the ultraviolet light U1 emitted from the excimer lamp 12 be incident onto the optical filter 25 with smaller incident angles, thereby less likely to decrease the light extraction efficiency.

Furthermore, the inactivation device 1 is provided with the reflective member 13 on the +Y side of the light-extraction surface 15. The reflective member 13 includes the reflective surface 13a made of a material that is reflective to the ultraviolet light U2 emitted from the light-extraction surface 15. In the present embodiment, the reflective surface 13a has a conical trapezoidal shape with a bottom surface 13b that is in contact with a support surface 43b described below, and the reflective surface 13a of the reflective member 13 is constituted by the outer side face of the reflective member 13. The reflective surface 13a is inclined with respect to the light-extraction surface 15, as shown in FIG. 23, reflecting the ultraviolet light U2 incident thereon to the outside of the enclosure 10.

When the reflective member 13 has the reflective surface 13a with the shape described above, the ultraviolet light U2 incident on the reflective surface 13a through the light-extraction surface 15 is reflected to be directly away from the enclosure 10 in the direction of the X-Z-plane (only the X direction is illustrated in FIG. 23).
As shown in FIG. 23, the traveling direction of the ultraviolet light U2 that has passed through the light-extraction surface 15 is changed by the reflective member 13, increasing the emission angle of the ultraviolet light U2. In other words, this configuration preferentially enables the ultraviolet light irradiation in the wide-angle direction, which has a large emission angle with respect to the light-extraction surface 15, thereby increasing the radiation intensity of ultraviolet light in the wide-angle direction.

In addition, as shown in FIG. 23, the inactivation device 1 of the present embodiment is provided with a light-shielding member 21 that blocks, of the ultraviolet light U2 that has been reflected by the reflective surface 13a of the reflective member 13, the traveling of the ultraviolet light U2 that travels in the -Y direction, in other words, closer toward a location in which the excimer lamps 12 are mounted. In this way, by providing the light-shielding member 21, which blocks the ultraviolet light U2 traveling toward the area where the excimer lamp 12 is mounted, in other words, the opposite of an area where the ultraviolet light irradiation is intended, this prevents irradiation to an area where ultraviolet light irradiation is undesired.

As shown in FIGS. 21 to 23, the light-shielding member 21 has a conical trapezoidal shape with a top surface (upper surface 21t) on which the enclosure 10 is mounted. The side face of the light-shielding member 21 is inclined with respect to the light-extraction surface 15 in a manner that the side face is open toward the outside of the enclosure 10. The area enclosed by the side face of the light-shielding member 21, i.e., the inside of the light-shielding member 21, is hollow, and when viewed from the +Y direction, the light-shielding member 21 is disposed to surround the outside of the reflective member 13.

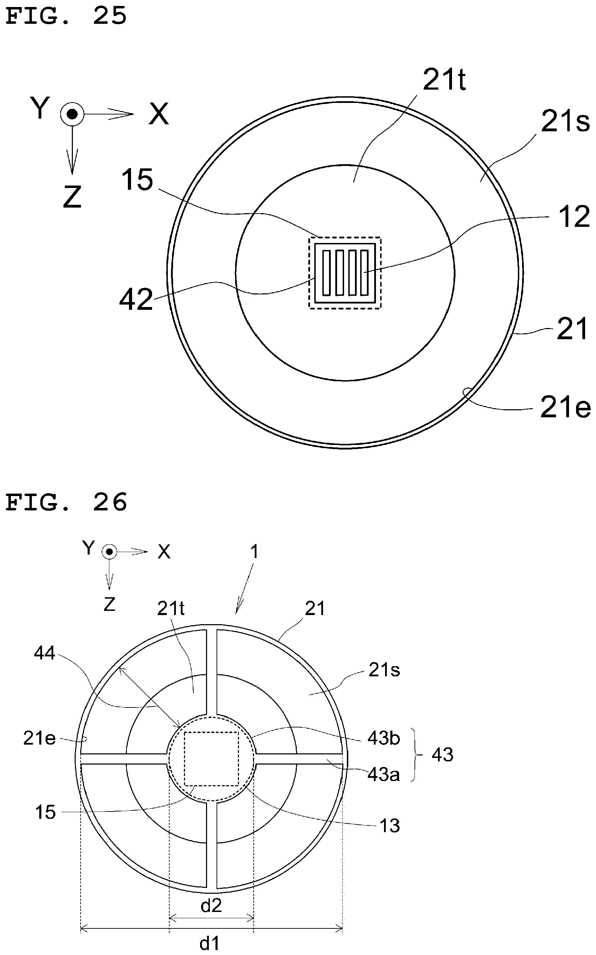

The light-shielding member 21 has a hole 42. This hole 42 will be explained using FIG. 25. FIG. 25 is a schematic view illustrating the relationship between the enclosure 10 and the light-shielding member 21 when viewed from the +Y direction without the reflective member 13, the window member 14, and a supporter 43 that will be described later. As shown in FIG. 25, the light-shielding member 21 is mounted such that the light-extraction surface 15 and the hole 42 overlap when viewed from the +Y direction.

In the present embodiment, the reflective member 13 is disposed on the support surface 43b (see FIG. 26) of the light-shielding member 21. FIG. 26 is a schematic view of the inactivation device 1 of the second embodiment when viewed from the +Y direction. As shown in FIG. 26, the light-shielding member 21 includes, as the components of the supporter 43, support rods 43a extending from the inner side face 21s of the light-shielding member 21 toward the reflective member 13, and a support surface 43b to which the support rods 43a are connected. The reflective member 13 is disposed on this support surface 43b. In addition, the light-shielding member 21 has openings 44 between the supporter 43 and an inner edge 21e of the light-shielding member 21. The ultraviolet light U2 reflected at the reflective surface 13a of the reflective member 13 passes through the openings 44 and between the reflective surface 13a and the inner side face 21s of the light-shielding member 21. As shown in FIG. 26, the enclosure 10 is disposed such that the openings 44 and the light-extraction surface 15 do not overlap when viewed from the +Y direction. The opening diameter d1 of the light-shielding member 21 may be twice or more as large as the diameter d2 of the bottom surface 13b of the reflective member 13 (see FIG. 26). The area of the openings of the light-shielding member 21 may be four times or more as large as the area of the bottom surface 13b of the reflective member 13. The material of the light-shielding member 21 can be selected in a manner similar to the first light-shielding member 22 described above in the first embodiment.

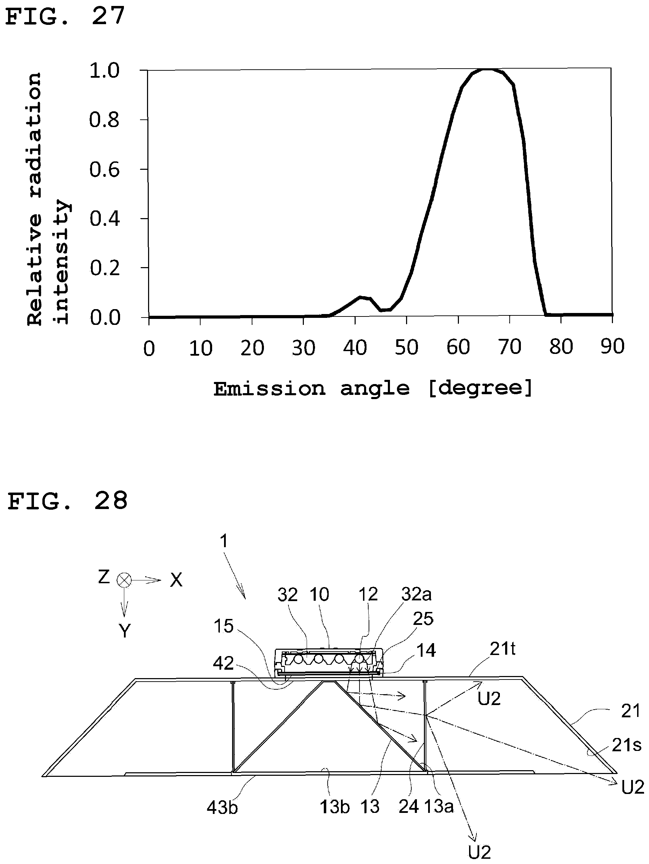

FIG. 27 is a graph indicating the simulation results of the relative radiation intensity with respect to the emission angle of ultraviolet light U2 emitted from the inactivation device 1 of the present invention.

### Simulation S4

The simulation S4 was performed with considering the configuration of the second embodiment shown in FIGS. 21 to 23. In the simulation S4, the four excimer lamps 12 were arranged in the enclosure 10. Specifically, each excimer lamp 12 had an approximately cylindrical shape with a diameter of 6 mm and a length of 70 mm. The shape of the reflective surface 13a of the reflective member 13 was configured to have a conical trapezoidal shape, and the reflective surface 13a was inclined by 45 degrees with respect to the light-extraction surface 15. Furthermore, the electrode blocks 32 between which the excimer lamp 12 is mounted have tapered reflective surfaces 32a inclined at 55 degrees thereon (see FIG. 24). The simulation was performed with considering that the optical filter 25 was set to have characteristics shown in FIG. 7.

The traveling direction of the ultraviolet light U2 is changed by the reflective member 13 (see FIG. 23), the effect of which will be described with reference to FIG. 27. As shown in FIG. 27, the relative radiation intensity of ultraviolet light indicates 0 when the emission angle is in a range from 0 degrees or more to less than 35 degrees, and the value of the relative radiation intensity of ultraviolet light rises when the emission angle exceeds 35 degrees. The relative radiation intensity of ultraviolet light gradually increases as the emission angle increases, becomes 0.1 exhibiting a local maximum at around 40 degrees, decreases at around 45 degrees, increases again at around 50 degrees, reaches a maximum value at around 65 degrees, and then gradually decreases to 0 at approximately 75 degrees (see FIG. 27). In other words, the reflective member 13 changes the emission angle of the ultraviolet light U2 to a wide-angle direction, thus resulting in the irradiation of ultraviolet light in the range from 50 degrees to 75 degrees. Hence, this configuration makes the illuminance in the area irradiated with ultraviolet light in the wide-angle direction higher than the illuminance in the area irradiated with ultraviolet light in the 0 degrees direction.

Although the results of the simulation S4 in FIG. 27 were calculated under the above conditions, it is understood that the configuration according to the present invention makes the radiation intensity of ultraviolet light in the wide-angle direction higher than that in the 0 degrees direction, even if the conditions such as the size ratio and the number of the excimer lamps differ from the above conditions.

### Variation examples

Hereinafter, the variation examples of the second embodiment will be described with reference to the drawings.

### First variation example

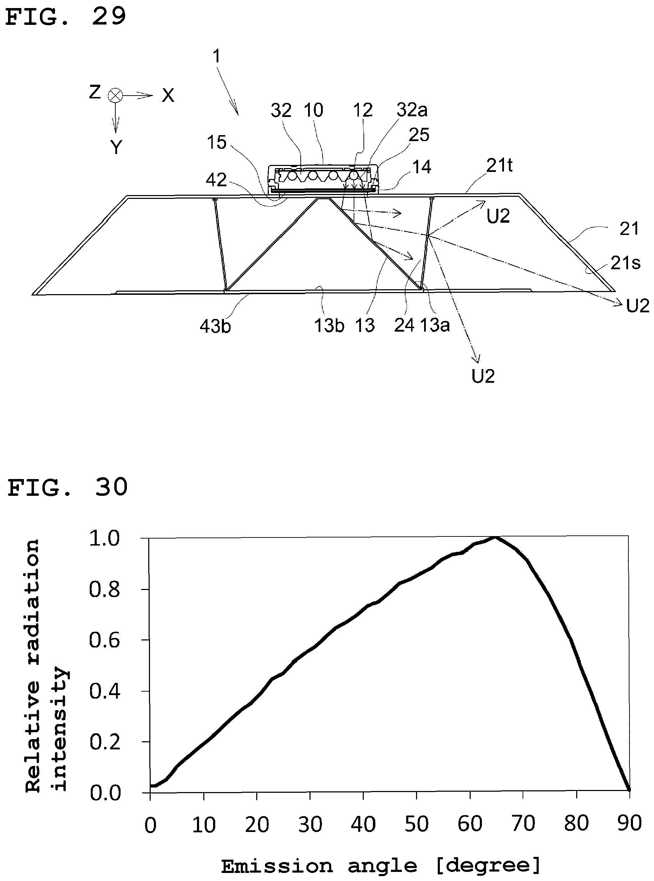

The diffusion member 24 can be added to the configuration of the second embodiment. FIG. 28 is a plan view of the first variation example of the second embodiment, as is similar to FIG. 23, such that the cross-sectional view thereof obtained by cutting in a plane perpendicular to the light-extraction surface 15 is viewed in the Z-direction. As shown in FIG. 28, the diffusion member 24 is disposed on the outside of the reflective member 13, i.e., in a manner to surround the reflective surface 13a. As shown in FIG. 29, the diffusion member 24 can be disposed to be inclined with respect to the light-extraction surface 15. Disposing the diffusion member 24 in this manner allows the traveling direction of the ultraviolet light U2 that has passed through the light-extraction surface 15 to be changed by the reflective member 13 and then diffused by the diffusion member 24. In other words, the first variation example serves the effects similar to those of the inactivation device 1 of the first embodiment described above with reference to FIGS. 8 to 11.

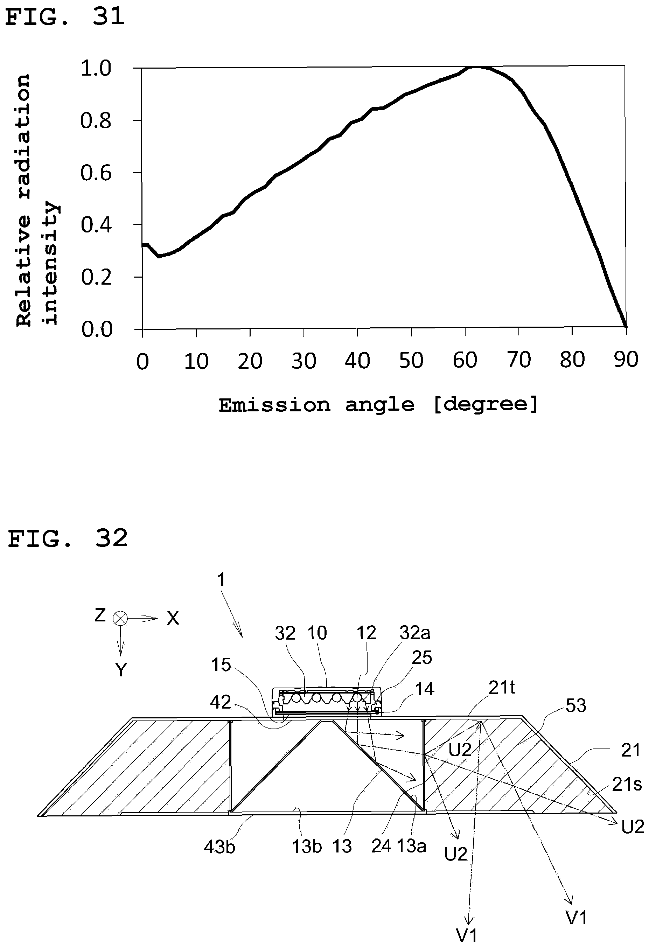

FIGS. 30 and 31 are graphs indicating the result of a simulation S5 in which the relative radiation intensity of the first variation example of the second embodiment was calculated with respect to the emission angle of ultraviolet light.

### Simulation S5

The simulation S5 was performed with considering the configuration of the first variation example of the second embodiment shown in FIGS. 28 and 29. In the simulation S5, the diffusion member 24 is disposed to surround the outside of the reflective surface 13a of the reflective member 13. The simulations each were performed for the case in which the diffusion member 24 is disposed perpendicular to the light-extraction surface 15 and for the case in which the diffusion member 24 is inclined at 5 degrees with respect to the light-extraction surface 15. Other conditions are the same as those in the simulation S4.

FIG. 30 corresponds to the results of simulation S5 for the case in which the diffusion member 24 is disposed perpendicular to the light-extraction surface 15, and FIG. 31 corresponds to the results of simulation S5 for the case in which the diffusion member 24 is disposed to be inclined with respect to the light-extraction surface 15. In FIGS. 30 and 31, the discussion similar to that for the first variation example and the second variation example of the first embodiment can be made in that the relative radiation intensity in the wide-angle direction is higher than the relative radiation intensity in the 0 degrees direction, and in that providing the diffusion member 24 reduces the difference in the radiation intensity at each emission angle. Furthermore, when FIG. 30 and FIG. 31 are compared with each other, the relative radiation intensity in the narrow-angle direction, i.e., in the range from 0 degrees to 20 degrees, is increased. In this way, when the diffusion member 24 is disposed to be inclined with respect to the light-extraction surface 15, it is possible to change the relative radiation intensity with respect to the emission angle, thereby capable of achieving the design suitable for the mounting location of the inactivation device 1 and the irradiation area of ultraviolet light.

### Second variation example

In addition to the configuration of the first variation example, a phosphor layer 53 that converts the ultraviolet light incident thereon to visible light can be formed on the surface of the light-shielding member 21 on which the ultraviolet light U2 is incident. FIG. 32 is a plan view of the second variation example of the second embodiment, as is similar to FIG. 23, such that the cross-sectional view thereof obtained by cutting in a plane perpendicular to the light-extraction surface 15 is viewed in the Z-direction. In FIG. 32, the area where the phosphor layer 53 is formed is hatched.

As shown in FIG. 32, the traveling direction of the ultraviolet light U2 that has passed through the light-extraction surface 15 is changed by the reflective member 13 and then diffused by the diffusion member 24. Part of the ultraviolet light U2 that has been diffused by the diffusion member 24 is incident on the phosphor layer 53 formed on the inner surface of the light-shielding member 21. The ultraviolet light U2 incident on the phosphor layer 53 is converted into the visible light V1. Forming the phosphor layer 53 in this way enables the ultraviolet light U2 to be used more effectively than the case in which the light-shielding member 21 simply blocks the ultraviolet light U2; therefore, the inactivation device 1 can be used to perform ultraviolet light irradiation, and at the same time, can be utilized as an illumination device.

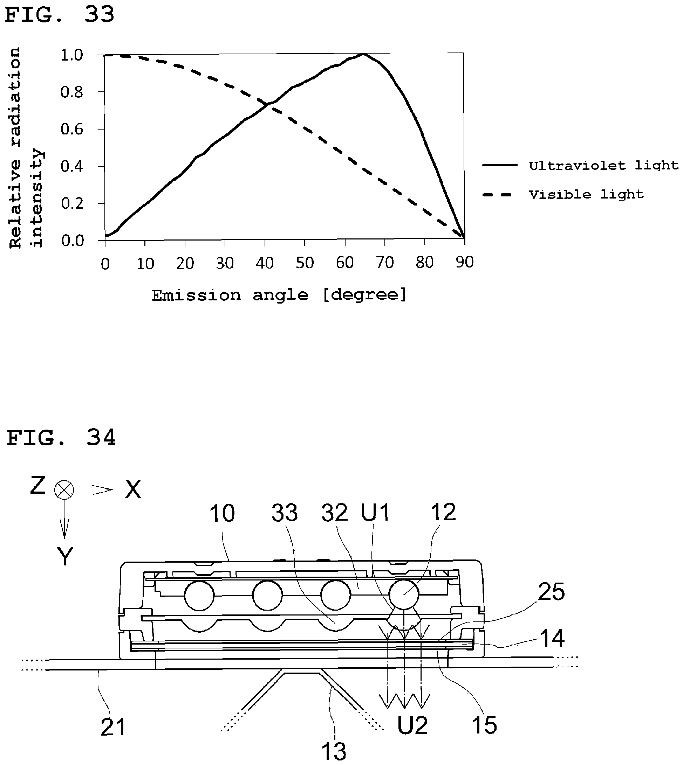

The effect of forming the phosphor layer 53 on the inner surface of the light-shielding member 21 will be described using FIG. 33. FIG. 33 is a graph indicating the results of a simulation S6 in which the relative radiation intensity of ultraviolet light of the second variation example of the second embodiment was calculated with respect to the emission angle thereof.

### Simulation S6

The simulation S6 has a condition different from that of the simulation S5 in that the phosphor layer 53 is formed on the surface of the light-shielding member 21 where ultraviolet light U2 is incident (see FIG. 32), and the other conditions are the similar to those of the simulation S5 in which the diffusion member 24 is disposed perpendicular to the light-extraction surface 15.

FIG. 33 shows the relative radiation intensity of ultraviolet light and visible light with respect to the emission angle, respectively. The relative radiation intensity of ultraviolet light in the second variation example of the second embodiment is the same as that in the first variation example (see FIG. 30). In contrast, the relative radiation intensity of the visible light V1 shows a maximum of 1 in the 0 degrees directions and gradually decreases to reach 0 at 90 degrees. The phosphor layer 53 converts the ultraviolet light U2 incident thereon into the visible light V1, but the visible light V1 emitted from the phosphor layer 53 has no directivity. Hence, as is similar to the described above, the relative radiation intensity in the 0 degrees direction, which is perpendicular to the light-extraction surface 15, is higher than the relative radiation intensity in the wide-angle direction. Therefore, the inactivation device 1 can be used to perform ultraviolet light irradiation over a wide area, and simultaneously, can be utilized as a visible light illumination device.

### Other variation examples

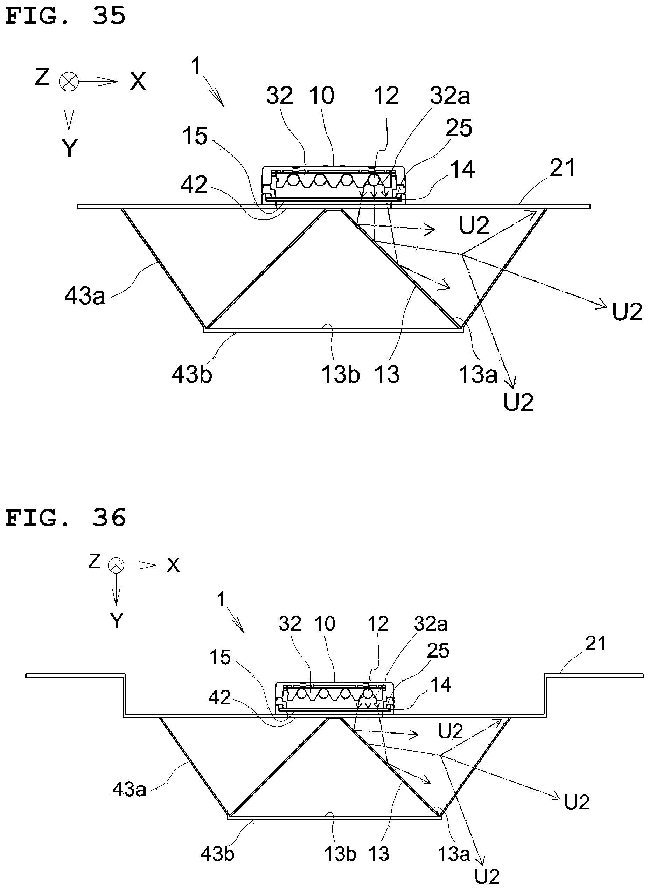

Hereinafter, other variation examples of the inactivation device 1 of the second embodiment will be described.
<1>
   The same discussion can be made as that in the first embodiment. In other words, even in the present embodiment, if the control to limit the irradiation of ultraviolet light having wavelengths considered harmful to human bodies is not necessary, the inactivation device 1 need not necessarily be provided with the optical filter 25. Also, if the excimer lamp 12 and the light-extraction surface 15 are close in the Y direction, the electrode block 32 need not necessarily have the tapered reflective surface 32a.
<2>
   FIG. 34 is a conceptual view illustrating an example in a cylindrical lens 33 is used as an optical control member. Even if the electrode block 32 does not have the tapered reflective surface 32a, an optical lens such as the cylindrical lens 33 can be used, as shown in FIG. 34, in order to make ultraviolet light U1 emitted from the excimer lamp 12 be incident at smaller incident angles to the light-extraction surface 15.
<3>
   In the present embodiment, the light-shielding member 21 is described as having the hole 42 in the upper surface thereof. This is provided for the purpose of securing a light path that guides the ultraviolet light U1 emitted from the excimer lamp 12, which is mounted at a position on the -Y side with respect to the light-shielding member 21, and to the reflective member 13. From this viewpoint, when the enclosure 10, which houses the excimer lamps 12, is mounted at a position on the +Y side with respect to the upper surface of the light-shielding member 21, in other words, inside the light-shielding member 21, the hole 42 is not necessarily needed.
<4>
   In the second embodiment, its first variation example and second variation example, the reflective member 13 is described as being disposed on the supporter 43 provided in the light-shielding member 21; however, the reflective member 13 may be, for example, fixed to the enclosure 10 using another member. In addition, the reflective member 13 may not have a conical trapezoidal shape; for example, it can have a conical shape or a square pyramid shape. Furthermore, a plurality of the reflective members 13 can also be arranged.
<5>
   In the second embodiment, its first variation example and second variation example, the light-shielding member 21 is described as having the side wall including the inner side face 21s; however, it can be a flat plate having its main plane in the X-Z plane without such side wall, as shown in FIG. 35, for example. In addition, as shown in FIG. 36, part of the light-shielding member 21 may be configured to be at a position on the -Y side with respect to the enclosure 10. In this case, the light-shielding member 21 may be fixed to a mounting surface, such as a ceiling, for example, thus the inactivation device 1 may be mounted on the ceiling or the like.

When the light-shielding member is a flat plate, the light-shielding member 21 is desired to extend its area over the opening area of the light-extraction surface 15. Specifically, when the center of a circle having an area four times or more as large as the opening area of the light-extraction surface 15 is disposed at the center of the light-extraction surface 15, the light-shielding member 21 is desirably configured such that the area of the light-shielding member 21 is larger than or equal to the area of the opening of the light-extraction surface 15.

### Other embodiments

Hereinafter, the configuration of other embodiments will be described below.
<1>
The following effects are expected by adopting the configuration including the optical filter 25 and the optical lens represented by the collimator lens 31 in the inactivation device 1 described above. Although the device having this configuration is fundamentally highly suitable for the use for the purpose of inactivating bacteria and/or viruses, it does not exclude its use for other purposes. For this reason, such a device will hereinafter be referred to as an "ultraviolet light irradiation device".

This ultraviolet light irradiation device includes an ultraviolet light source (LED element 11, excimer lamp 12, etc.) that emits the ultraviolet light U1 having a main emission wavelength belonging to within the wavelength range from 190 nm or more to less than 240 nm, and an enclosure 10 that includes the light-extraction surface 15 constituted by an opening or a window member and that houses the ultraviolet light source thereinside. Furthermore, this ultraviolet light irradiation device includes the optical filter 25 that transmits ultraviolet light, of the ultraviolet light U1 emitted from the ultraviolet light source, having a wavelength belonging to within the range from 190 nm or more to less than 240 nm, and suppress the transmission of ultraviolet light having a wavelength in the range from 240 nm or more to less than 300 nm. As a result, ultraviolet light emitted from the ultraviolet light source is radiated to the outside through the optical filter.

Furthermore, the ultraviolet light irradiation device includes an optical lens represented by the collimator lens 31 between the ultraviolet light source (e.g., LED element 11, excimer lamp 12, etc.) and the optical filter 25. The optical lens converts light flux emitted from the ultraviolet light source into collimated light. As a result, the ultraviolet light U1 emitted from the ultraviolet light source is likely to be incident on the optical filter 25 at smaller incident angles.

The optical filter 25 is formed with, for example, a multilayer film that is constituted by multiple dielectric layers having different refractive indices, and that is designed to exhibit such a wavelength selectivity. However, the optical filter 25 has a property (incident angle dependency) in which its transmittance and reflectance of each wavelength vary depending on the incident angle of ultraviolet light incident thereon. As a typical example, increasing the incident angle with respect to the optical filter 25 decreases the transmittance in a wavelength range from 190 nm or more to less than 240 nm, and increases the reflectance. This suggests a possibility that, depending on the incident angle of the ultraviolet light with respect to the optical filter 25, the extraction efficiency of ultraviolet light to the outside of the ultraviolet light irradiation device is reduced for ultraviolet light having a wavelength band of 190 nm or more to less than 240 nm.

In contrast, the ultraviolet light irradiation device according to the configuration described above includes the optical lens, which reduces the divergence angle of the light flux emitted from the ultraviolet light source, between the ultraviolet light source (11, 12) and the optical filter 25. This makes ultraviolet light become easier to be incident on the optical filter at smaller incident angles, thus the light-extraction efficiency is less likely to decrease. The optical lens may have the function of converting the light flux emitted from the ultraviolet light source into collimated light, as is similar to the collimator lens 31.

When the plurality of LED elements 11 is provided as the ultraviolet light source, it is preferable that the plurality of optical lenses is also provided. Typically, it is suitable for each optical lens to be correspondingly arranged for each LED element 11. This allows the ultraviolet light emitted from each LED element 11 to be transmitted through the optical lens arranged at a position corresponding to the respective LED element 11 and to be guided to the optical filter, thus enabling most of the ultraviolet light to be incident at smaller incident angles with respect to the optical filter.

When the collimator lens 31 is used as an optical lens, each lens surface of the collimator lens 31 is arranged at a position corresponding to each LED element 11, reducing the divergence angle of the light flux emitted from each LED element 11. As described above, the collimator lens 31 typically converts the light flux emitted from each LED element into collimated light. In other words, the ultraviolet light U1 emitted from LED element 11 is collimated by the collimator lens 31 in the Y direction, and then is incident on the optical filter 25 at a small incident angle. After that, the ultraviolet light U2 that has passed through the optical filter 25, and that has a wavelength band of 190 nm or more to less than 240 nm, is extracted to the outside of the enclosure 10. Note that the collimator lens 31 itself can be a single lens array as long as each lens is arranged at a position corresponding to the respective LED element 11.
<2>
The configuration provided in the inactivation device 1 described above is merely an example, and the present invention is not limited to each of the configurations shown in the figures.

Applicable product of the present invention can provide the germicidal capabilities and capabilities of inactivating viruses, which are inherent in ultraviolet light, without causing erythema or keratitis on the skin or eyes of people or animals, by using an ultraviolet light source that emits ultraviolet light having an emission wavelength belonging to within the wavelength range from 190 nm or more and less than 240 nm. In particular, unlike conventional ultraviolet light sources, the ultraviolet light source is characterized to be used in a space where people and animals can enter, hence mounting it in indoor or outdoor manned environments can irradiate the entire environment with the ultraviolet light, inactivating viruses in the space and on the surfaces of components mounted in the environment.

This addresses Goal 3 of the UN-led Sustainable Development Goals (SDGs): "Ensuring healthy lives and promote welfare for all people of all ages" and contributes significantly to Target 3.3 "By 2030, end the epidemics of AIDS, tuberculosis, malaria and neglected tropical diseases and combat hepatitis, water-borne diseases, and other communicable diseases".

### Reference Signs List

- 1: Inactivation device
- 10: Enclosure
- 11: LED element
- 12: Excimer lamp
- 13: Reflective member
- 13a: Reflective surface of reflective member
- 13t: Upper surface of reflective member
- 13b: Bottom surface of reflective member
- 14: Window member
- 15: Light-extraction surface
- 21: Light-shielding member
- 21t: Upper surface of light-shielding member
- 21s: inner side face of light-shielding member
- 21e: inner edge of light-shielding member
- 22: First light-shielding member
- 22s: Side wall of first light-shielding member
- 23: Second light-shielding member
- 23t: Upper surface of second light-shielding member
- 23s: Side portion of second light-shielding member
- 23b: Bottom portion of second light-shielding member
- 24: Diffusion member
- 25: Optical filter
- 31: Collimator lens
- 32: Electrode block
- 32a: Reflective surface of electrode block
- 33: Cylindrical lens
- 41: Mounting member
- 42: Hole
- 43: Supporter
- 43a: Support rod
- 43b: Support surface
- 44: Opening
- 51: Visible light source
- 52: Visible light illumination
- 53: Phosphor layer
- A1: First area
- A2: Second area
- C1: Curve
- D1: Width
- d1: Opening diameter
- d2: Diameter
- U1, U2: Ultraviolet light
- V1: Visible light

## Claims

1. An inactivation device (1) for bacteria and/or viruses comprising:
an ultraviolet light source emitting ultraviolet light having a main emission wavelength belonging to within a wavelength range from 190 nm or more to less than 240 nm;
an enclosure (10) including a light-extraction surface (15) that is constituted by an opening or a window member (14), and housing the ultraviolet light source thereinside; and
a reflective member (13) on which the ultraviolet light that has passed through the light-extraction surface (15) is incident and changes a traveling direction of the ultraviolet light,
wherein the reflective member (13) has a reflective surface that makes an optical axis of the ultraviolet light that has been incident change to a direction directly away from the enclosure (10), or to a direction away from the enclosure (10) after traveling toward a center area of the enclosure (10), with respect to a first direction parallel to the light-extraction surface (15).

2. The inactivation device (1) for bacteria and/or viruses according to claim 1, further comprising a light-shielding member (21) that, of the ultraviolet light reflected on the reflective surface of the reflective member (13), blocks at least part of the ultraviolet light traveling toward the ultraviolet light source with respect to a second direction orthogonal to the light-extraction surface (15).

3. The inactivation device (1) for bacteria and/or viruses according to claim 2, further comprising a diffusion member that diffuses the ultraviolet light in an optical path of the ultraviolet light reflected by the reflective surface.

4. The inactivation device (1) for bacteria and/or viruses according to any one of claims 1 to 3, further comprising an optical control member that directs the ultraviolet light emitted from the ultraviolet light source in a second direction, and that is disposed between the light-extraction surface (15) and the ultraviolet light source in an optical path of the ultraviolet light emitted from the ultraviolet light source.

5. The inactivation device (1) for bacteria and/or viruses according to claim 4, further comprising an optical filter that is provided between the ultraviolet light source and the reflective member (13), wherein the optical filter transmits ultraviolet light having a wavelength belonging to within a wavelength range from 190 nm or more to less than 240 nm, and suppresses a transmission of ultraviolet light having a wavelength in a range from 240 nm or more to less than 300 nm.

6. The inactivation device (1) for bacteria and/or viruses according to any one of claims 1 to 3, the reflective surface includes a side face of a member having a conical shape, a frustum shape, or a curved rotational body shape.

7. The inactivation device (1) for bacteria and/or viruses according to claim 1, wherein the ultraviolet light source includes a plurality of LED elements; and
the inactivation device (1) for bacteria and/or viruses further comprising, when viewed from a second direction orthogonal to the light-extraction surface (15), a first area with a relatively low arrangement density or no arrangement of the LED elements, and a second area with a relatively higher arrangement density of the LED elements than that of the first area, and the second area being arranged to surround the first area.

8. The inactivation device (1) for bacteria and/or viruses according to claim 7, wherein the first area is an area where no LED element is disposed, and the light-extraction surface (15) is a surface facing the second area when viewed in the second direction and has an annular shape.

9. The inactivation device (1) for bacteria and/or viruses according to claim 7 or 8, further comprising:
a first light-shielding member (22) that blocks, of the ultraviolet light reflected on the reflective surface of the reflective member (13), at least part of the ultraviolet light traveling toward the ultraviolet light source with respect to the second direction; and
a second light-shielding member (23) that blocks at least part of the ultraviolet light traveling away from the ultraviolet light source with respect to the second direction.

10. The inactivation device (1) for bacteria and/or viruses according to claim 9, wherein the reflective member (13) is arranged to surround the first area when viewed from the second direction.

11. The inactivation device (1) for bacteria and/or viruses according to claim 10,
further comprising a diffusion member that diffuses the ultraviolet light that has been reflected on the reflective surface,
wherein the reflective surface is formed to make the optical axis of the ultraviolet light incident thereon change in a direction directly away from the enclosure (10) with respect to the first direction, and
the diffusion member is arranged to surround the outside of the reflective member (13) when viewed from the second direction.

12. The inactivation device (1) for bacteria and/or viruses according to claim 10,
further comprising a diffusion member that diffuses the ultraviolet light that has been reflected on the reflective surface,
wherein the reflective surface is formed to make the optical axis of the ultraviolet light incident thereon change in a direction away from the enclosure (10) after traveling toward the center area of the enclosure (10) in the first direction, and
the diffusion member is arranged to surround the inside of the reflective member (13) when viewed from the second direction.

13. The inactivation device (1) for bacteria and/or viruses according to claim 1, wherein the ultraviolet light source includes an excimer lamp,
the inactivation device (1) for bacteria and/or viruses further comprising a light-shielding member (21) that, of the ultraviolet light reflected on the reflective surface of the reflective member (13), blocks at least part of the ultraviolet light traveling toward the ultraviolet light source with respect to the second direction orthogonal to the light-extraction surface (15), and
wherein the light-shielding member (21) is arranged to surround the reflective member (13) when viewed from the second direction.

14. The inactivation device (1) for bacteria and/or viruses according to claim 13, further comprising a diffusion member that diffuses the ultraviolet light that has been reflected on the reflective surface, and wherein the diffusion member is arranged to surround the outside of the reflective member (13) when viewed from the second direction.

15. The inactivation device (1) for bacteria and/or viruses according to claim 13 or 14, wherein the light-shielding member (21) includes an opening through which ultraviolet light emitted from the light-extraction surface (15) passes, and the enclosure (10) is arranged such that the opening and the light-extraction surface (15) do not overlap when viewed from the second direction.

16. The inactivation device (1) for bacteria and/or viruses according to claim 13 or 14, wherein the light-shielding member (21) includes a supporter that supports the reflective member (13), and the ultraviolet light transmits between the supporter and the inner side face of the light-shielding member (21).

17. The inactivation device (1) for bacteria and/or viruses according to claim 2, further comprising a phosphor layer that converts the ultraviolet light incident thereon into visible light and that is formed on at least part of the surface of the light-shielding member (21) that blocks a traveling of the ultraviolet light.
